# EUROPEAN PATENT APPLICATION

(11) **EP 4 299 746 A1**
(43) Date of publication of application: **03.01.2024**
(21) Application number: 21935490.9
(22) Date of filing: 06.04.2021
(51) Int. Cl.: C12N 15/55, C12N 9/14, G01N 33/48, C12Q 1/6869, C12M 1/34

(54) **MODIFIED PRP43 HELICASE AND USE THEREOF**

(71) Applicant: Qitan Technology Ltd., Chengdu, Sichuan 610041 (CN)
(72) Inventor: ZHANG, Zhougang, Chengdu, Sichuan 610041 (CN); LI, Wen, Chengdu, Sichuan 610041 (CN); WANG, Yanshuang, Chengdu, Sichuan 610041 (CN); WANG, Muyang, Chengdu, Sichuan 610041 (CN)
(74) Representative: Vossius & Partner Patentanwälte Rechtsanwälte mbB
(86) International application number: PCT/CN2021/085609
(87) International publication number: WO 2022/213253

(57) **Abstract**

The present application relates to a modified Prp43 helicase and use thereof. The Prp43 helicase has enhanced ATP hydrolytic or unwinding activity due to introduction of mutations and/or introduction of an auxiliary protein, and can remain binding to the target polynucleotide for a long period of time, thereby allowing the enzyme to control the rate of movement of the polynucleotide continuously and stably at a suitable rate required for sequencing. Thus, the modified or engineered Prp43 helicase mutant of the present application allows for the control of movement of a target polynucleotide in a more advantageous manner, which can be used for nanopore sequencing.

## Description

### TECHNICAL FIELD

The present application relates to nucleic acid sequencing technology.

### BACKGROUND

Nanopore sequencing is the third-generation nucleic acid sequencing technology that obtains DNA/RNA sequence information by recording different electrical signals generated by different bases of a DNA/RNA strand as it passes through a nanopore. One of the challenges of the nanopore sequencing technology is that the DNA/RNA molecule often passes through the nanopore too quickly, exceeding the resolution of the instrument, and thus it is difficult to obtain accurate electrical signals reflecting sequence information. Thus, how to control or reduce the speed of the DNA/RNA molecule passing through the nanopore is crucial to improving the accuracy of nanopore sequencing technology.

Currently, an emerging method for characterizing a polynucleotide includes contact and interaction of a transmembrane pore, a helicase and the polynucleotide, whereby the helicase controls movement of a target polynucleotide to pass through a nanopore to increase the residence time of the polynucleotide at the nanopore. For example, patents WO2013057495A3 and US20150191709A1 disclose a novel method for characterizing a target polynucleotide, wherein a pore and a Hel308 helicase or a molecular motor capable of binding to nucleotides in the target polynucleotide are used. The helicase or molecular motor described in the above invention can effectively control movement of the target polynucleotide to pass through the pore. In addition, patents US20150065354A1 and US9617591B2 disclose a method for characterizing a target polynucleotide using an XPD helicase, wherein a pore and an XPD helicase are utilized. The XPD helicase described in the above invention can control movement of the target polynucleotide to pass through the pore. In addition, patents US 20160257942A1 and US20180179500 A1 disclose that a T4 phage-derived Dda helicase and certain homologous proteins thereof can be applied to the sequencing of a polynucleotide passing through a pore.

In nature, helicases can be divided into six superfamilies (SFs), in which helicases of the SF1 and SF2 superfamilies perform translocation and unwinding functions in the form of a monomer, and the SF3-SF6 families function in the form of a polymer. In the application of nanopore sequencing, the helicase acting in the form of a monomer is easier to apply and has a more homogeneous performance. Helicases of the SF1 and SF2 superfamilies are classified into different families based on properties such as protein sequence homology, domain arrangement, substrate binding form and specificity, unwinding polarity (5'-3' and 3'-5' directions), and mechanisms of unwinding or translocation. The SF1 superfamily includes helicases of the UvrD/Rep family, helicases of the Upf1-like family, and helicases of the Pif1-like family; the SF2 superfamily includes helicases of the Rad3/XPD family, helicases of the Ski2-like family, helicases of the DEAH/RHA family, helicases of the NS3/NPH-II family, helicases of the DEAD-Box family, helicases of the RIG-I-like family, helicases of the RecQ-like family, helicases of the RecG-like family, helicases of the Swi/Snf family, and helicases of the T1R family. Dda helicases derived from RecD and T4 phage belong to the Pif1-like family of the SF1 superfamily, with a single-stranded DNA as the preferred substrate, and perform translocation and unwinding in the 5'-3' direction, and common helicases belonging to this family also include Pif1 helicase, TrwC helicase, and the like; a Hel308 helicase derived from the *Methanococcoides burtonii* strain (as disclosed in US20150191709A1) is a helicase of the ski2-like family of the SF2 superfamily, which can perform polar translocation or unwinding on a double-stranded nucleic acid in the 3'-5' direction using a single-stranded DNA or RNA as a substrate, and common helicases belonging to this family also include ski2 helicase, Brr2 helicase, Mtr4 helicase, and the like; an XPD helicase is a helicase of the Rad3/XPD family of the SF2 superfamily, which specifically binds to a single-stranded DNA, and performs polar translocation or unwinding on a double-stranded nucleic acid in the 5'-3' direction, and common helicases belonging to this family also include Rad3 helicase and the like.

Although various helicases that can be used in the nanopore sequencing technology are disclosed in the prior art, each helicase has its advantages and disadvantages and its applicable environment, and these helicases still have difficulty meeting the more rigorous requirements of scientific research and medical technology, etc., for nucleic acid sequencing technologies in many aspects. Therefore, there is still a need for a novel helicase that can be used in the nucleic acid nanopore sequencing technology in order to improve the applicability, accuracy, sensitivity, etc., of the nanopore sequencing technology.

### SUMMARY

The inventors have found that a Prp43 helicase, especially a modified Prp43 helicase, can control movement of a polynucleotide molecule to pass through a nanopore, and thus can be used in the nanopore sequencing technology.

Thus, a first aspect of the present application relates to a modified Prp43 helicase, which comprises a RecA1 domain, a RecA2 domain, and a Ratchet domain, wherein the modified Prp43 helicase comprises insertion or replacement of 1, 2, 3, 4, 5, 6, 7, 8, 9, 10, 11, or more cysteines and/or insertion or replacement of 1, 2, 3, 4, 5, 6, 7, 8, 9, 10, 11, or more non-natural amino acids introduced into at least one domain selected from the RecA1 domain, the RecA2 domain, or the Ratchet domain, relative to a corresponding wild-type Prp43 helicase or a fragment thereof.

A second aspect of the present application relates to a protein construct, which comprises the modified Prp43 helicase according to the first aspect of the present application, and a G-Path domain of an auxiliary activator protein Pfal or a fragment of Pfal containing the G-Path domain fused to the C-terminus or N-terminus of the Prp43 helicase.

A third aspect of the present application relates to a nucleic acid encoding the modified Prp43 helicase according to the first aspect of the present application or the protein construct according to the second aspect of the present application.

A fourth aspect of the present application relates to an expression vector comprising the nucleic acid according to the third aspect of the present application.

A fifth aspect of the present application relates to a host cell comprising the nucleic acid according to the third aspect of the present application or the expression vector according to the fourth aspect of the present application. A sixth aspect of the present application relates to a method for preparing the protein construct according to the second aspect of the present application, which comprises: providing a polypeptide of SEQ ID NO: 1 or a variant thereof and a polypeptide of SEQ ID NO: 26 or a variant thereof, introducing at least one cysteine residue and/or at least one non-natural amino acid into the polypeptide of SEQ ID NO: 1 or the variant thereof, and fusing the polypeptide of SEQ ID NO: 26 or the variant thereof to the C-terminus or N-terminus of the resulting polypeptide to form the protein construct.

A seventh aspect of the present application relates to a method for preparing the modified Prp43 helicase according to the first aspect of the present application or the protein construct according to the second aspect of the present application, which comprises: culturing the host cell according to the fifth aspect of the present application, inducing expression, and purifying the resulting expression product.

An eighth aspect of the present application relates to a method for controlling movement of a polynucleotide molecule, which comprises contacting the polynucleotide molecule with the modified Prp43 helicase according to the first aspect of the present application or the protein construct according to the second aspect of the present application.

A ninth aspect of the present application relates to a method for characterizing a target polynucleotide, which comprises:
(a) contacting the target polynucleotide with the modified Prp43 helicase according to the first aspect of the present application or the protein construct according to the second aspect of the present application, such that the Prp43 helicase or protein construct controls movement of the target polynucleotide to pass through a nanopore; (b) acquiring one or more characteristics of nucleotides in the target polynucleotide when interacting with the nanopore, thereby characterizing the target polynucleotide.

A tenth aspect of the present application relates to use of the modified Prp43 helicase according to the first aspect of the present application or the protein construct according to the second aspect of the present application for characterizing a target polynucleotide or controlling movement of a target polynucleotide to pass through a pore. An eleventh aspect of the present application relates to an analysis device for characterizing a target polynucleotide, wherein the analysis device comprises one or more nanopores, one or more modified Prp43 helicases according to the first aspect of the present application or protein constructs according to the second aspect of the present application, and one or more containers.

A twelfth aspect of the present application relates to a method for forming a sensor for characterizing a target polynucleotide, which comprises providing a nanopore, and forming a complex between the nanopore and the modified Prp43 helicase according to the first aspect of the present application or the protein construct according to the second aspect of the present application.

The present application provides a novel Prp43 helicase mutant or a construct thereof that can be used for nucleic acid nanopore sequencing, which has enhanced ATP hydrolytic or unwinding activity due to introduction of mutations and/or introduction of an auxiliary protein, and/or can remain binding to a target polynucleotide for a long period of time, thereby allowing for continuous and stable control of the rate of movement of the polynucleotide. Thus, the Prp43 helicase mutant or the construct thereof of the present application can continuously control the movement of a target polynucleotide to pass through a pore at a suitable rate required for sequencing, thereby improving the throughput and accuracy of nanopore sequencing.

### DETAILED DESCRIPTION

### Definitions

In order to more clearly explain the embodiments of the present invention, some scientific terms and proper names are used herein. Unless explicitly defined herein, all such terms and names should be understood to have the meanings as commonly understood by those skilled in the art. For greater clarity, the following definitions are made for certain terms used herein.

The term "polypeptide" refers to a molecule comprising more than five amino acid residues linked by peptide bonds. The polypeptide may typically comprise 20 or more amino acids, preferably 50 or more amino acids, or 100 or more amino acids. Herein, the terms "protein" and the term "polypeptide" are considered to have the same meaning; thus, the terms "protein" and "polypeptide" are used interchangeably. The polypeptide may optionally be modified (e.g., glycosylated, phosphorylated, acylated, famesylated, isopentenylated, sulfonated, etc.) to increase its functionality or activity. A polypeptide that exhibits activity in the presence of a particular substrate under certain conditions may be referred to as an "enzyme". It should be understood that due to the degeneracy of genetic codes, a variety of nucleotide sequences encoding a given polypeptide may be generated. "Nucleic acid" described herein is a generic term for deoxyribonucleic acid (DNA) and ribonucleic acid (RNA), and is a biomacromolecular compound formed by polymerizing a plurality of nucleotide monomers. Herein, the term "nucleic acid" and the term "polynucleotide" are considered to have the same meaning; thus, the term "nucleic acid" and the term "polynucleotide" are used interchangeably.

The nucleotide monomer consists of pentose, a phosphate group, and a nitrogenous base. If the pentose is ribose, the polymer formed is RNA; if the pentose is deoxyribose, the polymer formed is DNA. Nitrogenous bases in nucleotides may include, but are not limited to: adenine (A), guanine (G), thymine (T), uracil (U), and cytosine (C). The nucleotides may be naturally occurring or synthetic. Thus, the "nucleotide" described herein includes, but is not limited to: adenosine monophosphate (AMP), guanosine monophosphate (GMP), thymidine monophosphate (TMP), uridine monophosphate (UMP), cytosine monophosphate (CMP), cyclic adenosine monophosphate (cAMP), cyclic guanosine monophosphate (cGMP), deoxyadenosine monophosphate (dAMP), deoxyguanosine monophosphate (dGMP), deoxythymidine monophosphate (dTMP), deoxyuridine monophosphate (dUMP), and deoxycytidine monophosphate (dCMP). Preferably, the nucleotide is selected from AMP, TMP, GMP, CMP, UMP, dAMP, dTMP, dGMP, and dCMP.

In the present application, a "fragment" of a polypeptide or polypeptide domain refers to a polypeptide or polypeptide domain that has a deletion of one or more (e.g., several, several dozen, 100, etc.) amino acid residues at the amino and/or carboxyl terminus of the polypeptide or polypeptide domain, but still retains the desired activity. For example, a fragment of a Prp43 helicase refers to a polypeptide sequence that has a deletion of one or more (e.g., 1-5, 1-10, 1-20, 1-50, 1-100, 1-150, 1-200, or, e.g., 20, 30, 40, 50, 60, 70, 80, or 90) amino acid residues at the amino and/or carboxyl terminus of wild-type Prp43, but still retains helicase activity.

Typically, a fragment of a polypeptide or domain is of a length that is at least 50%, 55%, 60%, 65%, 70%, 75%, 80%, 85%, 90%, 92%, 94%, 96%, 98%, or 99% of the length of its original sequence. In the present application, a fragment of a polypeptide or domain comprises at least 50 amino acids, such as at least 60 amino acids, at least 70 amino acids, at least 80 amino acids, at least 90 amino acids, at least 100 amino acids, at least 150 amino acids, at least 200 amino acids, at least 250 amino acids, at least 300 amino acids, at least 350 amino acids, at least 400 amino acids, at least 500 amino acids, at least 650 amino acids, or at least 700 amino acids, depending on the length of the original polypeptide or domain. In the present application, a fragment of a polypeptide or domain may also comprise less than 700 amino acids, such as less than 600 amino acids, less than 500 amino acids, less than 400 amino acids, less than 300 amino acids, less than 200 amino acids, or less than 100 amino acids.

The term "expression" includes any step involved in the production of a polypeptide, including, but not limited to, transcription, post-transcriptional modification, translation, post-translational modification, and secretion.

An "expression vector" comprises a polynucleotide encoding a polypeptide that is operably linked to appropriate control sequences (e.g., a promoter, and transcriptional and translational termination signals) for *in vitro* expression and/or translation. The expression vector may be any vector (e.g., a plasmid or virus) that can be conveniently subjected to recombinant DNA procedures and can result in the expression of the polynucleotide. A vector is typically selected depending on the compatibility of the vector with the cell into which the vector is to be introduced. The vector may be a linear or a closed circular plasmid. The vector may be an autonomously replicating vector, i.e., a vector that exists as an extrachromosomal entity, is replicated independent of chromosomal replication, and is, e.g., a plasmid, an extrachromosomal element, a minichromosome, or an artificial chromosome. Alternatively, the vector may be a vector that, when introduced into a host cell, is integrated into the genome and replicated together with the chromosome into which has been integrated. The cloning vector to be integrated may be integrated at a random or predetermined target locus in the chromosome of the host cell. The vector system may be a single vector or plasmid or two or more vectors or plasmids that together contain the total DNA to be introduced into the genome of the host cell, or a transposon.

The term "control sequence" used herein refers to a component involved in the regulation of the expression of a coding sequence in a particular organism, either *in vivo* or *in vitro.* Examples of control sequences include a transcription initiation sequence, a termination sequence, a promoter, a leader sequence, a signal peptide, a propeptide, a prepropeptide, or an enhancer sequence; a Shine-Delgamo sequence, a repressor or activator sequence; an effective RNA processing signal, such as splicing and polyadenylation signals; a sequence that stabilizes cytoplasmic mRNA; a sequence that enhances translation efficiency (e.g., a ribosome binding site); a sequence that enhances protein stability; and a sequence that enhances protein secretion when desired.

"Host cell" defined herein refers to an organism that is suitable for genetic manipulation and may be used to produce a target product (e.g., the Prp43 helicase described herein). The host cell may be a host cell found in nature or a host cell derived from a parent host cell after genetic manipulation or classical mutagenesis. Advantageously, the host cell is a recombinant host cell. The host cell may be a prokaryotic, archaeal or eukaryotic host cell. The prokaryotic host cell may be, but is not limited to, a bacterial host cell. The eukaryotic host cell may be, but is not limited to, a yeast, fungal, amoeba, algae, plant, animal, or insect host cell.

The term "recombinant", when used with respect to a nucleic acid or protein (or enzyme), means that the nucleic acid or protein (or enzyme) has been subjected to sequence modification by human intervention as compared to its natural form. The term "recombinant", when referring to a cell (e.g., a host cell), means that the genome of the cell has been subjected to sequence modification by human intervention if compared to its natural form. Herein, the terms "recombinant" and "modified" are considered synonymous.

The term "replacement", when used with respect to a modified polypeptide or enzyme, means that a natural amino acid residue present in the corresponding wild-type polypeptide or enzyme is replaced with another amino acid residue. Herein, the terms "amino acid replacement" and "amino acid substitution" are considered synonymous.

The terms "variant" and "mutant" used herein have the same meaning and are used interchangeably. They may refer to polypeptides or nucleic acids. The variant refers to replacement, insertion, deletion, truncation, transversion, etc., at one or more positions relative to a reference sequence (typically the wild-type form of the nucleic acid or polypeptide). The variant can be generated by, for example, site saturation mutagenesis, scanning mutagenesis, insertional mutagenesis, random mutagenesis, site-directed mutagenesis, and directed evolution, as well as various other recombination methods known to those skilled in the art. Variant genes of nucleic acids can be artificially synthesized by techniques known in the art.

"Mature polypeptide" is defined herein as a polypeptide that is in its final form and is obtained after translation of mRNA into the polypeptide and post-translational modification of the polypeptide. Post-translational modifications include N-terminal processing, C-terminal truncation, glycosylation, phosphorylation, and removal of leader sequences (such as signal peptides and/or propeptides) by cleavage.

The similarity between two polypeptide sequences or nucleic acid sequences can be expressed in terms of their homology. Herein, "identity" and "homology" between two sequences are considered to have the same meaning and are used interchangeably herein. To determine the percentage of sequence homology or sequence identity between two amino acid sequences or two nucleic acid sequences, the sequences are aligned for the best match. The sequence identity is the percentage of identical matches between the two sequences over the aligned region. The percentage of sequence homology between two amino acid sequences or two polynucleotide sequences can be determined using well-known algorithms, such as the Needleman and Wunsch algorithms for aligning two sequences (Needleman, S. B. and Wunsch, C. D. (1970) J. Mol. Biol. 48, 443-453). This can be done, for example, by using the NEEDLE program from the EMBOSS program package. Those skilled in the art will understand that slightly different results may be produced when different algorithms or different parameters of a particular algorithm are used, but the percent identity between the two sequences does not vary significantly. "Opening" described herein refers to an opening of a polynucleotide-binding domain of the wild-type Prp43 helicase, or refers to an opening of a polynucleotide-binding portion binding to the Prp43 helicase. The opening is an opening that enables the polynucleotide to dissociate from the Prp43 helicase, and it may not be present at all times, but at least in one conformational state comprises at least one opening. "Modified Prp43 helicase" or construct comprising the modified Prp43 helicase described herein contains one or more openings. The modified Prp43 helicase enables two or more portions of the same monomer of the helicase to be connected to reduce the size of the opening.

"Above one", "at least one", "one or more", or "one or more than one" described herein includes: one, two, three, four, five, six, seven, eight, nine, ten, eleven, twelve, or more, etc.

"Above two", "two or more", or "two or more than two" described herein includes: two, three, four, five, six, seven, eight, nine, ten, eleven, twelve, or more, etc.

"Multiple" described herein includes, but is not limited to: three, four, five, six, seven, eight, nine, ten, eleven, twelve, or more, etc.

"And/or" described herein includes one listed item and any number of combinations of items.

"Include", "contain", or "comprise" described herein is an open-ended description that indicates the inclusion of the specified components or steps as described, as well as other specified components or steps that do not have a substantial effect. In particular, when the above terms are used to describe a sequence of a protein or nucleic acid, it is meant that the protein or nucleic acid may be composed of the sequence, or may have additional amino acid residues or nucleotides at one or both ends of the protein or nucleic acid, but still has the activity described herein (e.g., its ability to control the movement of a polynucleotide, etc.).

### Prp43 Helicase

The Prp43 helicase is a known helicase, the structure and function of which have been studied and reported in the prior art. See, for example, Marcel J. Tauchert et al., "Structural and functional analysis of the RNA helicase Prp43 from the thermophilic eukaryote Chaetomium thermophilum",Acta Cryst., 2016, F72, 112-120. However, there have been no reports of using Prp43 helicase for nanopore sequencing or for controlling movement of a polynucleotide molecule to pass through a nanopore.

The Prp43 helicase is a DEAH/RHA helicase of the SF2 superfamily, which can perform translocation or unwinding on a double-stranded DNA or RNA nucleic acid in the 3'-5' direction on the basis of single-stranded DNA or RNA, and the helicases belonging to this family also include Prp22 helicase, Prp2 helicase, MLE helicase, DHX9 helicase, and the like.

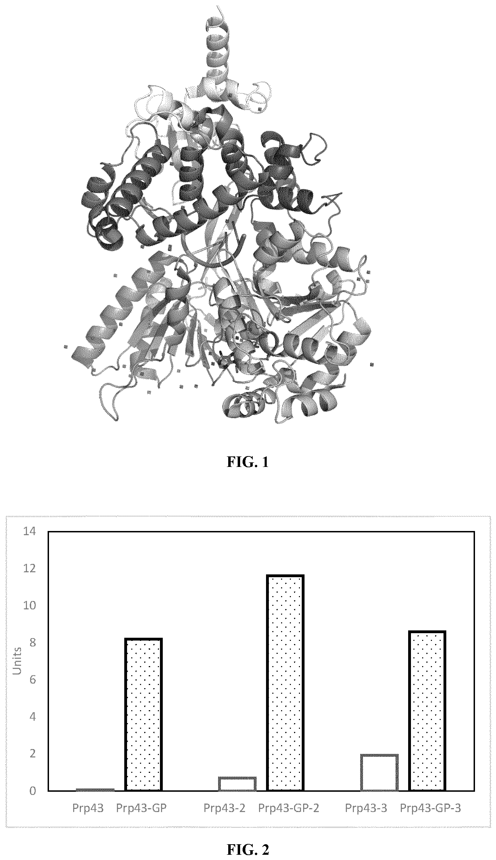

The 3D structure of Prp43 helicase and its component domains have been illustrated in the prior art. For example, FIG. 1 is a schematic diagram of a 3D structure of a Prp43 helicase (SEQ ID NO: 1) derived from *Chaetomium thermophilum.* In addition to two core domains of RecA1 (P97-R273) and RecA2 (T274-T458), the Prp43 helicase contains several domains: N-terminal domain (M1-L96), C-terminal WH domain (Y459-P526), Ratchet domain (L527-V640), OB domain (S641-A764), and the like. RecA1 and RecA2 contain 7 conserved motifs, of which Ia (TQPRRVAA), Ib (TDGQLLR), and IV (LLFLTG) interact with a substrate nucleic acid, motifs I (GSGKT), II (DEAH), V (TNIAETSLT), and VI (QRAGRAGR) are involved in nucleotide binding, and motif III (SAT) is associated with hydrolysis of nucleotides and translocation or unwinding of nucleic acids.

The Prp43 helicase is rich in positively charged amino acids at the top of the RecA1 and RecA2 domains, which form a channel around single-stranded DNA or RNA together with the WH, Ratchet, and OB domains at the C-terminus.

The inventors found that although the Prp43 helicase has a strong affinity for single-stranded DNA or RNA, the process is a thermodynamic equilibrium process, and cannot completely control movement of a target nucleic acid to pass through a pole, especially when the target nucleic acid is long, such as 1000 bases in length, 5000 bases in length, 10000 bases in length, 100000 bases in length, or more bases in length. The inventors found that it is possible to ensure the binding of the enzyme to the nucleic acid and to continuously control the nucleic acid to pass through the nanopore by modifying the Prp43 helicase. Specifically, the inventors found that it is possible to reduce the opening size of the polynucleotide-binding domain of the Prp43 helicase by introducing one or more cysteines or non-natural amino acids into the RecA 1, RecA2, and/or Ratchet domains of the Prp43 helicase, thereby improving the binding ability of its target nucleic acid.

Thus, a first aspect of the present application relates to a modified Prp43 helicase, which comprises a RecA1 domain, a RecA2 domain, and a Ratchet domain, wherein the modified Prp43 helicase comprises insertion or replacement of 1, 2, 3, 4, 5, 6, 7, 8, 9, 10, 11, or more cysteines and/or insertion or replacement of 1, 2, 3, 4, 5, 6, 7, 8, 9, 10, 11, or more non-natural amino acids introduced into at least one domain selected from the RecA1 domain, the RecA2 domain, or the Ratchet domain, relative to a corresponding wild-type Prp43 helicase or a fragment thereof.

Preferably, at least one cysteine residue and/or at least one non-natural amino acid can be introduced into any one of the following groups:
(a) a RecA1 domain;
(b) a RecA2 domain;
(c) a Ratchet domain;
(d) a RecA1 domain and a Ratchet domain; and
(e) a RecA2 domain and a Ratchet domain.
non-natural amino acids described herein include, but are not limited to: 4-azido-L-phenylalanine (Faz), 4-acetyl-L-phenylalanine, 3-acetyl-L-phenylalanine, 4-acetoacetyl-L-phenylalanine, O-allyl-L-tyrosine, 3-(phenylselenoalkyl)-L-alanine, O-2-propyn-1-yl-L-tyrosine, 4-(dihydroxyboryl)-L-phenylalanine, 4-[(ethylsulfanyl)carbonyl]-L-phenylalanine, (2S)-2-amino-3-{4-[(propan-2-ylsulfanyl)carbonyl]phenyl}propanoic acid, (2S)-2-amino-3-{4-[(2-amino-3-sulfanylpropanoyl)amino]phenyl}propanoic acid, O-methyl-L-tyrosine, 4-amino-L-phenylalanine, 4-cyano-L-phenylalanine, 3-cyano-L-phenylalanine, 4-fluoro-L-phenylalanine, 4-iodo-L-phenylalanine, 4-bromo-L-phenylalanine, O-(trifluoromethyl)tyrosine, 4-nitro-L-phenylalanine, 3-hydroxy-L-tyrosine, 3-amino-L-tyrosine, 3-iodo-L-tyrosine, 4-isopropyl-L-phenylalanine, 3-(2-naphthyl)-L-alanine, 4-phenyl-L-phenylalanine, (2S)-2-amino-3-(naphthalen-2-ylamino)propionic acid, 6-(methylsulfanyl)norleucine, 6-oxo-L-lysine, D-tyrosine, (2R)-2-hydroxy-3-(4-hydroxyphenyl)propionic acid, (2R)-2-aminooctanoate 3-(2,2'-bipyridin-5-yl)-D-alanine, 2-amino-3-(8-hydroxy-3-quinolinyl)propionic acid, 4-benzoyl-L-phenylalanine, S-(2-nitrobenzyl)cysteine, (2R)-2-amino-3-[(2-nitrobenzyl)sulfanyl]propionic acid, (2S)-2-amino-3-[(2-nitrobenzyl)oxy]propanoic acid, O-(4,5-dimethoxy-2-nitrobenzyl)-L-serine, (2S)-2-amino-6-({[(2-nitrobenzyl)oxy]carbonyl}amino)hexanoic acid, O-(2-nitrobenzyl)-L-tyrosine, 2-nitrophenylalanine, 4-[(E)-phenyldiazenyl]-L-phenylalanine, 4-[3-(trifluoromethyl)-3H-diaziridin-3-yl]-D-phenylalanine, 2-amino-3-[[5-(dimethylamino)-1-naphthyl]sulfonylamino]propanoic acid, (2S)-2-amino-4-(7-hydroxy-2-oxo-2H-chromen-4-yl)butanoic acid, (2S)-3-[(6-acetylnaphthalen-2-yl)amino]-2-aminopropionic acid, 4-(carboxymethyl)phenylalanine, 3-nitro-L-tyrosine, O-thio-L-tyrosine, (2R)-6-acetylamino-2-aminocaproate, 1-methylhistidine, 2-aminononanoic acid, 2-aminodecanoic acid, L-homocysteine, 5-sulfanyl norvaline, 6-sulfanyl-L-norleucine, 5-(methylsulfanyl)-L-norvaline, N6-{[(2R,3R)-3-methyl-3,4-dihydro-2H-pyrrol-2-yl]carbonyl}-L-lysine, N6-[(benzyloxy)carbonyl]lysine, (2S)-2-amino-6-[(cyclopentylcarbonyl)amino]hexanoic acid, N6-[(cyclopentyloxy)carbonyl]-L-lysine, (2S)-2-amino-6-{ [(2R)-tetrahydrofuran-2-ylcarbonyl]amino}hexanoic acid, (2S)-2-amino-8-[(2R,3S)-3-ethynyltetrahydrofuran-2-yl]-8-oxooctanoic acid, N6-(tert-butoxycarbonyl)-L-lysine, (2S)-2-hydroxy-6-({[(2-methyl-2-propyl)oxy]carbonyl}amino)hexanoic acid, N6-[(allyloxy)carbonyl]lysine, (2S)-2-amino-6-({[(2-azidobenzyl)oxy]carbonyl}amino)hexanoic acid, N6-L-prolyl-L-lysine, (2S)-2-atnino-6-{[(prop-2-yn-1-yloxy)carbonyl]amino}hexanoic acid, or N6-[(2 azidoethoxy)carbonyl]-L-lysine. "Prp43 helicase" described herein should be understood in its broadest sense and is considered to encompass homologous proteins of the Prp43 helicase (e.g., SEQ ID NO: 1). Typically, an enzyme is considered to be a Prp43 helicase as long as it has DNA/RNA unwinding activity, contains a RecA1 domain, a RecA2 domain, and/or a Ratchet domain, and has at least 30% homology, e.g., at least 35%, at least 40%, at least 50%, at least 60%, at least 70%, at least 80%, at least 85%, at least 90%, at least 91%, at least 92%, at least 93%, at least 94%, at least 95%, at least 96%, at least 97%, at least 98%, at least 99%, or at least 99.9% homology, to SEQ ID NO: 1. Thus, some helicases (such as those listed in Table 1) referred to as HrpA helicases or HrpB helicases are also considered to fall within the scope of the "Prp43 helicase" of the present application.

The Prp43 helicase described herein may be any conventionally derived Prp43 helicase, for example, the Prp43 helicase may be derived from *Chaetomium thermophilum, Bathycoccus prasinos*, *Uncultured bacterium*, *Archaeon*, *Parcubacteria*, *Sorangium cellulosum*, *Candidatus Sungbacteria*, *Mycolicibacterium chitae*, *Parcubacteria*, *Thermodesulforhabdus norvegica*, *Deltaproteobacteria*, *Puniceicoccales*, *Desulfobacterium vacuolatum*, or *Desulfobacter sp*.*,* or derived from viral metagenomes and the like. Some examples of homologous Prp43 helicases that can be used in the present application are shown in Table 1, but the Prp43 helicases of the present application are not limited to these examples.

**Table 1: examples of ctPrp43 homologous proteins**

| **SEQ ID** | **Name** | **Function** | **Source** | **Lengths** | **Identities** | **NCBI number** |
|---|---|---|---|---|---|---|
| SEQ ID NO: 1 | ctPrp43 | ATP-dependent RNA helicase | Chaetomium thermophilum | 764 | 100% | XP_006691112. 1 |
| SEQ ID NO: 2 | bpPrp43 | ATP-dependent RNA helicase | Bathycoccus prasinos | 711 | 64.69% | XP_007511797. 1 |
| SEQ ID NO: 3 | unPrp43 | ATP-dependent RNA helicase | uncultured bacterium | 533 | 59% | L7RXK7 |
| SEQ ID NO: 4 | vmPrp43 | ATP-dependent RNA helicase | viral metagenome | 635 | 53.18% | QHU07681.1 |
| SEQ ID NO: 5 | arPrp43 | ATP-dependent RNA helicase | archaeon | 628 | 41.90% | A0A482T3B5 |
| SEQ ID NO: 6 | paPrp43 | ATP-dependent RNA helicase | Parcubacteria group bacterium GW2011 | 740 | 41.81% | A0A0G1FX53 |
| SEQ ID NO: 7 | scHrpA | ATP-dependent RNA helicase | Sorangium cellulosum | 1230 | 40.25% | A0A150RF04 |
| SEQ ID NO: 8 | csHrpA | ATP-dependent RNA helicase | Candidatus Sungbacteria bacterium | 767 | 39.76% | A0A1G2K8K0 |
| SEQ ID NO: 9 | mcHrpA | ATP-dependent RNA helicase | Mycolicibacteriu m chitae | 1311 | 39.61% | A0A3S4TQL2 |
| SEQ ID NO: 10 | paHrpB | ATP-dependent RNA helicase | Parcubacteria group bacterium ADurb.Bin305 | 1305 | 39.42% | A0A1V5RPG5 |
| SEQ ID NO: 11 | tnHrpA | ATP-dependent RNA helicase | Thermodesulforh abdus norvegica | 703 | 38.14% | A0A7C0WSW3 |
| SEQ ID NO: 12 | dbHrpA | ATP-dependent RNA helicase | Deltaproteobacter ia bacterium | 1277 | 38.06% | A0A661LIX4 |
| SEQ ID NO: 13 | pbHrpA | ATP-dependent RNA helicase | Puniceicoccales bacterium CK1056 | 1255 | 37.95% | A0A6B2LXC5 |
| SEQ ID NO: 14 | dvHrpA | ATP-dependent RNA helicase | Desulfobacterium vacuolatum DSM 3385 | 1331 | 36.99% | A0A1W2CQF0 |
| SEQ ID NO: 15 | dsHrpA | ATP-dependent RNA helicase | Desulfobacter sp. | 1311 | 36.67% | A0A357YTE1 |

| | | | | | | |
|---|---|---|---|---|---|---|
| Most preferably, the Prp43 helicase described herein is derived from *Chaetomium thermophilum.* | | | | | | |

Thus, in some preferred embodiments, the present application provides a modified Prp43 helicase, which comprises a variant of SEQ ID NO: 1 or a fragment thereof, wherein the variant comprises insertion or replacement of 1, 2, 3, 4, 5, 6, 7, 8, 9, 10, 11, or more cysteines and/or insertion or replacement of 1, 2, 3, 4, 5, 6, 7, 8, 9, 10, 11, or more non-natural amino acids introduced into the RecA1 domain, the RecA2 domain, and/or the Ratchet domain.

In some preferred embodiments, the variant comprises introduction of at least one cysteine residue and/or at least one non-natural amino acid residue at positions corresponding to any one or two or more ofM157, Q161, D165, F181, E182, N183, R324, L328, E332, R335, L351, P352, P353, H354, D321, E320, R358, P563, A564, N565, D603, K605, K606, H609, Y615, R616, S619, N623, A626, or K630 of SEQ ID NO: 1. More preferably, the introduced cysteine residues or non-natural amino acid residues are located at positions corresponding to any one or two or more of F181, P352, S619, or N623 of SEQ ID NO: 1.

It has been found that removal of the N-terminal domain of the wild-type Prp43 helicase is more advantageous in controlling movement of nucleotides. Thus, in some preferred embodiments, the modified Prp43 helicase comprises removal of an N-terminal domain, preferably removal of at least 96, at least 90, at least 80, at least 70, at least 60, at least 50, at least 40, or at least 30 residues beginning at position 1 of the N-terminus. For SEQ ID NO: 1, M1-N60 is preferably removed, i.e., the fragment T61-A764 of SEQ ID NO: 1 is preferably used, and on this basis, insertion or replacement of one or more cysteines and/or insertion or replacement of one or more non-natural amino acids are introduced.

In order to improve the stability of binding of the Prp43 helicase described herein to the target polynucleotide and decrease the ability of the Prp43 helicase described herein to dissociate from the target polynucleotide, 2 or more cysteine residues or non-natural amino acid residues may be introduced, and an interconnection is formed between the introduced cysteines, between the introduced non-natural amino acids, between the introduced cysteines and non-natural amino acids, between the introduced cysteines and natural amino acids, or between the introduced non-natural amino acids and natural amino acids.

Preferably, any number and combination of two or more introduced cysteines may be connected to non-natural amino acids. For example, 2, 3, 4, 5, 6, 7, 8, or more cysteines and/or non-natural amino acids may be connected to each other. One or more cysteines may be connected to one or more cysteines. One or more cysteines may be connected to one or more non-natural amino acids, such as Faz. One or more non-natural amino acids such as Faz may be connected to one or more non-natural amino acids such as Faz. One or more cysteines may be connected to natural amino acids on one or more helicases. One or more non-natural amino acids such as Faz may be connected to natural amino acids on one or more helicases.

Preferably, the connection may be any connection, including temporary connection or permanent connection, such as covalent connection, hydrogen bonding connection, electrostatic interaction, π-π interaction, or hydrophobic interaction. In another specific embodiment of the present invention, the connection may be permanent, e.g., covalent connection. The covalent connection may be performed using a chemical cross-linking agent, which can vary in length from one carbon (carbonyl chloride linker) to many angstroms. The cross-linking agent includes, for example, maleimide, active ester, succinimide, azide, alkane, alkene, alkyne (such as dibenzocyclooctynol (DIBO or DBCO), difluorocycloalkyne, and linear alkyne), and the like. For another example, the cross-linking agent includes linear molecules such as polyethylene glycol (PEG), polypeptide, polysaccharide, deoxyribonucleic acid (DNA), peptide nucleic acid (PNA), threose nucleic acid (TNA), glycerol nucleic acid (GNA), saturated and unsaturated hydrocarbon, or polyamide, and catalysts such as TMAD, which may perform the connection through a -S-S bond.

In certain specific embodiments of the present invention, the TMAD catalyst is used to covalently connect cysteine residues introduced at positions F181 and N623 or at positions P352 and S619.

In some preferred embodiments, the modified Prp43 helicase further comprises replacement of one or more cysteine residues, preferably replacement of one or more cysteine residues corresponding to C148, C214, C303, C323, C377, C441, C508, C543, or C608 of SEQ ID NO: 1, and more preferably replacement of cysteine residues with an alanine, glycine, valine, isoleucine, leucine, phenylalanine, tyrosine, serine, threonine, aspartic acid, glutamic acid, lysine, arginine, histidine, methionine, tryptophan, glutamine, asparagine, or proline residue. In some preferred embodiments, to further confer the ability of the Prp43 helicase to control movement of a polynucleotide continuously and stably at a certain rate, the modified Prp43 helicase further comprises one or more amino acid modifications selected from the group consisting of:
(a) replacement of one or more amino acids that interact with nucleotides;
(b) replacement of one or more amino acids associated with binding of NTP and/or a divalent metal ion (e.g., Mg2+);
(c) replacement of one or more amino acids that interact with transmembrane pores; and
(d) further modification to reduce a negative charge on the surface of the Prp43 helicase.

Preferably, the amino acids that interact with the nucleotides and are replaced include, but are not limited to: amino acids corresponding to R152, R153, R180, T195, Q198, R201, E316, E317, G349, T381, N382, K403, K405, L416, P526, P557, R562, Q558, H688, P689, T708, K710, Y712, or R714 of SEQ ID NO: 1. Further preferably, at least one amino acid that interacts with phosphate groups of one or more nucleotides in the single-stranded DNA or RNA or double-stranded DNA or RNA is replaced.

Preferably, the one or more amino acids associated with the binding of NTP and/or a divalent metal ion (e.g., Mg²⁺) include, but are not limited to: amino acids corresponding to T126, D218, S387, E219, R432, R435, T121, K125, T127, T389, R162, D391, or F360 of SEQ ID NO: 1.

Preferably, the one or more amino acids that interact with transmembrane pores include, but are not limited to: amino acids corresponding to C303, E336, D288, R287, E286, E284, or E291 of SEQ ID NO: 1.

Further preferably, at least one amino acid that interacts with sugars and/or bases of one or more nucleotides in the single-stranded DNA or RNA or double-stranded DNA or RNA is substituted with an amino acid comprising a large side chain. The large side chain includes an increased number of carbon atoms, has increased length, has increased molecular volume, and/or has increased Van der Waals volume. The large side chain increases (i) electrostatic interaction, (ii) hydrogen bonding interaction, and/or (iii) cation-pi interaction between the at least one amino acid and one or more nucleotides in the single- or double-stranded DNA. The amino acid with the large side chain is not alanine (A), cysteine (C), glycine (G), selenocysteine (U), methionine (M), aspartic acid (D), or glutamic acid (E).

Preferably, the Prp43 helicase is further modified to reduce the negative charge on its surface. The Prp43 helicase further comprises replacement that increases the net positive charge. Preferably, the Prp43 helicase further comprises replacement or modification of an amino acid with a negatively charged surface or a polar or non-polar amino acid. Further preferably, the replacement comprises replacement of a negatively charged amino acid, an uncharged amino acid, an aromatic amino acid, or a polar or non-polar amino acid with a positively charged amino acid or an uncharged amino acid. The positively charged amino acid, uncharged amino acid, polar or non-polar amino acid, or aromatic amino acid may be a natural or non-natural amino acid, or specifically may be a synthetic or modified natural amino acid.

After modification, the Prp43 helicase described herein may have at least 30%, at least 40%, at least 50%, at least 60%, at least 70%, at least 80%, at least 85%, at least 90%, at least 91%, at least 92%, at least 93%, at least 94%, at least 95%, at least 96%, at least 97%, at least 98%, at least 99%, or at least 99.9% homology to the amino acid sequence of the corresponding wild-type Prp43 helicase.

In some more preferred embodiments, the Prp43 helicase is a variant of SEQ ID NO: 1 (i.e., derived from *Chaetomium thermophilum),* and the variant of SEQ ID NO: 1 comprises introduction of at least one cysteine residue and/or at least one non-natural amino acid at position F181 and/or N623 of SEQ ID NO: 1; or the variant of SEQ ID NO: 1 comprises introduction of at least one cysteine residue and/or at least one non-natural amino acid at position P352 and/or S619 of SEQ ID NO: 1.

In some more preferred embodiments, the Prp43 helicase is a variant of SEQ ID NO: 1 (i.e., derived from *Chaetomium thermophilum),* and the variant of SEQ ID NO: 1 further comprises replacement of at least one or more cysteines of SEQ ID NO: 1. The amino acid for the replacement may be alanine, glycine, valine, isoleucine, leucine, phenylalanine, tyrosine, serine, threonine, aspartic acid, glutamic acid, lysine, arginine, histidine, methionine, tryptophan, glutamine, asparagine, or proline. Preferably, the one or more replaced cysteines are C148, C214, C303, C323, C377, C441, C508, C543, or C608.

In some more preferred embodiments, the Prp43 helicase is a variant of SEQ ID NO: 1 (i.e., derived from *Chaetomium thermophilum),* and the variant of SEQ ID NO: 1 comprises removal of a sequence of M1-N60 of the N-terminal domain, and further preferably removal of a sequence of M1-L96 of the N-terminal domain. A specific embodiment of the present invention is a helicase comprising removal of a sequence of M1-N60 of the N-terminal domain.

In some more preferred embodiments, the Prp43 helicase is a variant of SEQ ID NO: 1 (i.e., derived from *Chaetomium thermophilum),* and the variant of SEQ ID NO: 1 has at least 30%, at least 40%, at least 50%, at least 60%, at least 70%, at least 80%, at least 85%, at least 90%, at least 91%, at least 92%, at least 93%, at least 94%, at least 95%, at least 96%, at least 97%, at least 98%, at least 99%, or at least 99.9% homology to the amino acid sequence of SEQ ID NO: 1. More preferably, the Prp43 helicase is a modified T61-A764 fragment of SEQ ID NO: 1.

In some more preferred embodiments, the Prp43 helicase is a modified T61-A764 fragment of SEQ ID NO: 1 (derived from *Chaetomium thermophilum),* and the modification is F181C/N623C/C508S or P352C/S619C/C508S.

In addition, the Prp43 helicase described herein can be modified to facilitate identification or purification, for example, to facilitate the secretion from a cell by adding a histidine residue (His-tag), an aspartic acid residue (asp-tag), streptavidin-tag, Flag-tag, SUMO-tag, GST-tag or MBP-tag, or by adding a signal sequence, wherein the polypeptide in the cell does not naturally contain the signal sequence. An alternative method for introducing a genetic tag is to attach the tag to a natural or artificial site on the Prp43 helicase by a chemical reaction.

The Prp43 helicase described herein may be in the form of a Prp43 helicase oligomer comprising one or more Prp43 helicases described herein.

In some embodiments, the Prp43 helicase oligomer may further comprise a wild-type Prp43 helicase or other types of helicases. The other types of helicases may include Hel308 helicase, XPD helicase, Dda helicase, RecD2 helicase, TraI helicase, TrwC helicase, etc.

Preferably, the Prp43 helicase and the wild-type Prp43 helicase, the Prp43 helicase and the Prp43 helicase, the wild-type Prp43 helicase and the wild-type Prp43 helicase, the Prp43 helicase and the other type of helicase, or the wild-type Prp43 helicase and the other type of helicase can be connected or arranged in a head-to-head, tail-to-tail, or head-to-tail manner.

Preferably, the Prp43 helicase oligomer comprises two or more Prp43 helicases described herein, wherein the Prp43 helicases may be different or identical.

### Protein Construct

Among physiological functions, the Prp43 helicase is involved in the dissociation of an intron spliceosome consisting of U2.U5.U6snRNPs during processing of pre-mRNA, in which the functioning of the enzyme requires interaction with two auxiliary proteins Ntr1 and Ntr2 containing glycine-rich motifs (G-Path motifs) to activate its ATP hydrolytic and unwinding activity; the Prp43 helicase is also involved in the ribosome synthesis process to help the maturation of the precursors of the 18S and 25S rRNAs, which also requires activation of the G-Path motif-rich proteins Pfal and Gno1.

The Prp43 helicase requires auxiliary proteins containing a G-Path domain to activate its ATP hydrolytic and unwinding activity under physiological functional conditions. Although this enzyme has weak activity in the absence of auxiliary proteins, it is more preferred that it has stronger ATP hydrolytic and unwinding activity in the presence of auxiliary activator proteins. In particular, the inventors have found that a single portion of a fragment of the auxiliary protein containing the G-Path domain still has an activating function.

Thus, in a second aspect of the present application, provided is a protein construct, which comprises the modified Prp43 helicase according to the first aspect of the present application, and a G-Path domain of an auxiliary activator protein Pfalor a fragment of Pfal containing the G-Path domain fused to the C-terminus or N-terminus of the Prp43 helicase. The protein construct may also be considered as a fusion protein.

In terms of the modified Prp43 helicase construct, since the G-Path domain of the auxiliary activator protein Pfal or the homologous protein thereof or the fragment containing the G-Path domain is fused to the C-terminus or N-terminus of the Prp43 helicase, the ATP hydrolytic and/or unwinding activity of the modified helicase is significantly enhanced, and the control of the movement of a nucleic acid to pass through a pole in the nanopore nucleic acid sequencing is facilitated.

In the protein construct, the number of modified Prp43 helicases may be one or more.

In the protein construct, the auxiliary activator protein Pfal may be a Pfal protein from various sources conventionally used in the art, and may be, for example, Pfal derived from *Chaetomium thermophilum var. thermophilum, Thermothielavioides terrestris, Thermothelomyces thermophilus, Podospora anserina, Neurospora tetrasperma, Coniochaeta sp., Monosporascus sp., Hypoxylon sp., Madurella mycetomatis,* or *Coniochaeta pulveracea.*

Some examples of homologous Pfa1 proteins that can be used in the Prp43 helicase construct of the present application are shown in Table 2, but the Pfal proteins of the present application are not limited to these examples.

**Table 2: ctPfa1 homologous proteins**

| **SEQ ID** | **Name** | **Source** | **Lengths** | **Identities** | **NCBI number** | **Note** |
|---|---|---|---|---|---|---|
| SEQ ID NO: 16 | ctPfa1 | Chaetomium thermophilum var. thermophilum DSM 1495 | 742 | 100% | XP_0066951 85 | hypothetical protein CTHT_0048220 |
| SEQ ID NO: 17 | ttnPfa1 | Thermothielavioides terrestris NRRL 8126 | 709 | 50.07%% | XP_0036541 07.1 | uncharacterized protein |
| SEQ ID NO: 18 | ttPfa1 | Thermothelomyces thermophilus ATCC 42464 | 700 | 47.44% | XP_0036654 22.1 | uncharacterized protein |
| SEQ ID NO: 19 | paPfa1 | Podospora anserina | 718 | 45.66% | XP_0019297 16.1 | uncharacterized protein |
| SEQ ID NO: 20 | ntPfa1 | Neurospora tetrasperma FGSC 2508 | 657 | 43.30% | XP_0098533 33.1 | hypothetical protein |
| SEQ ID NO: 21 | csPfa1 | Coniochaeta sp. 2T2.1 | 679 | 36.97% | KAB551879 4.1 | hypothetical protein |
| SEQ ID NO: 22 | maPfa1 | Monosporascus sp. GIB2 | 688 | 35.80% | RYP03421.1 | hypothetical protein |
| SEQ ID NO: 23 | hspfa1 | Hypoxylon sp. EC38 | 667 | 37.52% | OTA65903.1 | hypothetical protein |
| SEQ ID NO: 24 | mmPfa1 | Madurella mycetomatis | 682 | 45.19% | KXX74671.1 | Protein SQS1 |
| SEQ ID NO: 25 | cpPfa1 | Coniochaeta pulveracea | 718 | 36.95% | RKU41729.1 | squalene synthetase-like protein |

Preferably, the G-path domain sequence is a sequence of the Pfal auxiliary protein or the homologous protein thereof described above corresponding to a K662-G742 fragment (i.e., a sequence of SEQ ID NO: 26) of SEQ ID NO: 16 or a variant thereof.

In some preferred embodiments, in the protein construct, the amino acid sequence of the auxiliary activator protein Pfal is an amino acid sequence of SEQ ID NO: 16 or an amino acid sequence of a variant having at least 30%, at least 40%, at least 50%, at least 60%, at least 70%, at least 80%, at least 85%, at least 90%, at least 91%, at least 92%, at least 93%, at least 94%, at least 95%, at least 96%, at least 97%, at least 98%, at least 99%, or at least 99.9% homology to the amino acid sequence of SEQ ID NO: 16, and the auxiliary activator protein Pfa1 has a function of activating the Prp43 helicase.

In some more preferred embodiments, the G-Path domain of Pfa1 is the K662-G742 fragment of SEQ ID NO: 16.

In a most preferred embodiment, in the protein construct, the Prp43 helicase comprises a sequence of SEQ ID NO: 1 or a variant thereof, and the Pfal auxiliary activator protein comprises a sequence of SEQ ID NO: 16 or a variant thereof, or a G-Path domain sequence SEQ ID NO: 26 (corresponding to the K662-G742 fragment of the sequence of SEQ ID NO: 16) of SEQ ID NO: 16 or a variant thereof.

The protein construct described herein can be modified to facilitate identification or purification, for example, to facilitate the secretion from a cell by adding a histidine residue (His-tag), an aspartic acid residue (asp-tag), streptavidin-tag, Flag-tag, SUMO-tag, GST-tag, MBP-tag or Strep TagII-tag, or by adding a signal sequence, wherein the polypeptide in the cell does not naturally contain the signal sequence. An alternative method for introducing a genetic tag is to attach the tag to a natural or artificial site on the protein construct by a chemical reaction.

### Nucleic Acid

In a third aspect of the present application, provided is a nucleic acid encoding the Prp43 helicase according to the first aspect of the present application and/or the protein construct according to the second aspect of the present application.

### Expression Vector

In a fourth aspect of the present application, provided is an expression vector comprising the nucleic acid according to the third aspect of the present application. Preferably, the nucleic acid is operably linked to a regulatory element in the expression vector, wherein the regulatory element is preferably a promoter. In some specific embodiments of the present application, the promoter is selected from T7, trc, lac, ara, or λL. Preferably, the expression vector includes, but is not limited to, a plasmid, a virus, or a phage.

A variety of methods for inserting a nucleic acid into a nucleic acid construct or an expression vector are known to those skilled in the art. See, for example, Sambrook and Russell, Molecular Cloning: A Laboratory Manual, 3rd edition, CSHL Press, Cold Spring Harbor, NY, 2001.

### Host Cell

In a fifth aspect of the present application, provided is a host cell comprising the nucleic acid according to the third aspect of the present application or the expression vector according to the fourth aspect of the present application. Preferably, the host cell includes, but is not limited to, *Escherichia coli.* In one specific embodiment of the present application, the host cell is selected from BL21 (DE3), JM109 (DE3), B834 (DE3), TUNER, C41 (DE3), Rosetta2 (DE3), Origami, Origami B, or the like.

### Preparation Method for Prp43 Helicase or Protein Construct

A sixth aspect of the present application relates to a method for preparing the protein construct according to the second aspect of the present application, which comprises: providing a polypeptide of SEQ ID NO: 1 or a variant thereof and a polypeptide of SEQ ID NO: 26 or a variant thereof, introducing at least one cysteine residue and/or at least one non-natural amino acid into the polypeptide of SEQ ID NO: 1 or the variant thereof, and fusing the polypeptide of SEQ ID NO: 26 or the variant thereof to the C-terminus or N-terminus of the resulting polypeptide to form the protein construct.

A seventh aspect of the present application relates to a method for preparing the modified Prp43 helicase according to the first aspect of the present application or the protein construct according to the second aspect of the present application, which comprises: culturing the host cell according to the fifth aspect of the present application, inducing expression, and purifying the resulting expression product.

Genetic engineering techniques, such as overexpression of enzymes in host cells, genetic modification of host cells, or hybridization techniques, are known in the art, such as those described in Sambrook and Russel (2001) "Molecular Cloning: A Laboratory Manual" (3rd edition), Cold Spring Harbor Laboratory Press or F. Ausubel et al., "Current protocols in Molecular biology", Green Publishing and Wiley Interscience, New York (1987). For example, in one specific embodiment of the present application, the preparation method for the modified Prp43 helicase comprises: according to the amino acid sequence of the Prp43 helicase and/or the auxiliary activator protein or activator domain, obtaining a nucleic acid sequence encoding the Prp43 helicase, transforming the nucleic acid sequence to *Escherichia coli* after digestion and ligation to an expression vector, and inducing expression and purification to obtain the Prp43 helicase.

### Use of Prp43 Helicase or Protein Construct

The Prp43 helicase or the protein construct of the present application can be used to control movement of a polynucleotide molecule or to characterize a target polynucleotide.

The Prp43 helicase described herein is a useful tool for controlling movement of a target polynucleotide during strand sequencing, and when provided with conventional and necessary components to facilitate the movement, the Prp43 helicase moves in the 3'-5' direction along DNA or RNA, but the orientation of DNA or RNA in a pore (depending on which end of the DNA or RNA is captured) means that the Prp43 helicase can be used to move DNA or RNA into the pore against or along the direction of the applied field. By introducing a cysteine residue and/or at least one non-natural amino acid into a wild-type Prp43 helicase, the size or opening/closing of the opening of a polynucleotide-binding domain or polynucleotide-binding portion of the Prp43 helicase or construct, and the size or opening/closing of the opening through which the target polynucleotide is unwound, can be effectively reduced, thereby significantly decreasing the ability of the Prp43 helicase to dissociate from the target polynucleotide, and enhancing the ability to control the target polynucleotide to pass through the pore. By fusing a G-Path domain or a polypeptide containing the G-Path domain to the C-terminus or N-terminus of the wild-type Prp43 helicase or the modified Prp43 helicase, the ATP hydrolytic or unwinding activity of the modified Prp43 helicase can be effectively improved, thereby enhancing the ability to control the target polynucleotide to pass through the pore.

An eighth aspect of the present application relates to a method for controlling movement of a polynucleotide molecule, which comprises contacting the polynucleotide molecule with the modified Prp43 helicase according to the first aspect of the present application or the protein construct according to the second aspect of the present application.

Preferably, controlling movement of a polynucleotide is controlling movement of a polynucleotide to pass through a pore. The pore is a nanopore, and the nanopore is a transmembrane pore. The pore may be natural or artificial and includes, but is not limited to, a biological pore, a solid pore, or a pore in which a biological pore is hybridized with a solid pore. Preferably, the method may comprise controlling movement of a polynucleotide by one or more Prp43 helicases together.

A ninth aspect of the present application relates to a method for characterizing a target polynucleotide, which comprises:
(a) contacting the target polynucleotide with the modified Prp43 helicase according to the first aspect of the present application or the protein construct according to the second aspect of the present application, such that the Prp43 helicase or protein construct controls movement of the target polynucleotide to pass through a nanopore; (b) acquiring one or more characteristics of nucleotides in the target polynucleotide when interacting with the nanopore, thereby characterizing the target polynucleotide.

Preferably, steps (a) and (b) are repeated one or more times.

Preferably, any number of Prp43 helicases according to the present application may be used in the method.

Preferably, the number may be one or more, more preferably 1, 2, 3, 4, 5, 6, 7, 8, 9, or more. The two or more Prp43 helicases described herein may be identical or different. The wild-type Prp43 helicase or other types of helicases may also be comprised. Further, two or more helicases may be connected to each other or may be arranged only by separately binding to a polynucleotide, to exert a function of controlling the movement of the polynucleotide.

Preferably, the method further comprises the step of applying a potential difference across the pore in contact with the helicase or construct and the target polynucleotide.

Preferably, the pore is of a structure that allows hydrated ions to flow from one side of the membrane to the other side of the membrane driven by an applied potential. Further preferably, the pore is a nanopore, and the nanopore is a transmembrane pore. The transmembrane pore provides a channel for the movement of the target polynucleotide. Further preferably, the pore is selected from a biological pore, a solid pore, or a pore in which a biological pore is hybridized with a solid pore.

In some specific embodiments, the pore includes, but is not limited to, those derived from *Mycobacterium smegmatis* porin A, *Mycobacterium smegmatis* porin B, *Mycobacterium smegmatis* porin C, *Mycobacterium smegmatis* porin D, hemolysin, lysenin, interleukin, outer membrane porin F, outer membrane porin G, outer membrane phospholipase A, WZA, or *Neisseria* autotransporter lipoprotein, and the like.

The membrane may be any membrane present in the prior art, and is preferably an amphiphilic molecular layer, i.e., a layer formed by an amphiphilic molecule, such as phospholipid, having at least one hydrophilic portion and at least one lipophilic or hydrophobic portion, which may be synthetic or naturally occurring. Further preferably, the membrane is a bilayer lipid membrane. The target polynucleotide may be attached to the membrane by any known method. If the membrane is an amphiphilic molecular layer, such as a lipid bilayer, the polynucleotide is preferably attached to the membrane by a polypeptide present in the membrane or by a hydrophobic anchor present in the membrane. The hydrophobic anchor is preferably a lipid, fatty acid, sterol, carbon nanotube, or amino acid.

Preferably, when a force (e.g., a voltage) is applied across the pore, the rate at which the target polynucleotide passes through the pore is controlled by the Prp43 helicase or construct, so as to obtain an identifiable and stable current that can be used to characterize the target polynucleotide.

Preferably, the target polynucleotide is single-stranded, double-stranded, or at least partially double-stranded. Further preferably, the target polynucleotide can be modified by means of a tag, spacer unit, methylation, oxidation, or damage.

In one specific embodiment of the present application, the target polynucleotide is at least partially double-stranded. The double-stranded portion forms a Y-adaptor structure, and the Y-adaptor structure comprises a leader sequence that is threaded preferentially into the pore.

Further preferably, the target polynucleotide may be 10 to 100000 bases or more in length.

In one specific embodiment of the present application, the target polynucleotide may be at least 10, at least 50, at least 100, at least 200, at least 300, at least 400, at least 500, at least 1000, at least 2000, at least 5000, at least 10000, at least 50000, at least 100000 bases, or the like, in length.

Preferably, the helicase is incorporated into the internal nucleotides of the single-stranded polynucleotide. Preferably, the target polynucleotide is DNA or RNA.

Preferably, when the target polynucleotide is RNA, the RNA is modified to comprise non-RNA polynucleotides in order to enhance the ability and efficiency of the RNA to be sequenced to pass through the pore.

Preferably, the RNA modification comprises a step of ligating a DNA leader to the 3' end of the RNA to be sequenced. The RNA modification further comprises a step of reversely transcribing the RNA to be sequenced.

Preferably, the one or more characteristics are selected from the source, length, identity, sequence, or secondary structure of the target polynucleotide or whether the target polynucleotide is modified. Further preferably, the one or more characteristics are obtained by an electrical measurement and/or an optical measurement.

Further preferably, an electrical signal and/or an optical signal is generated by the electrical measurement and/or the optical measurement, wherein each nucleotide corresponds to a signal level, and then the electrical signal and/or the optical signal is converted into the characteristic of the nucleotide.

In one specific embodiment of the present application, the electrical measurement includes, but is not limited to, a current measurement, an impedance measurement, a tunnel measurement, a wind tunnel measurement, a field effect transistor (FET) measurement, or the like.

The electrical signal described herein is selected from measurement values of a current, voltage, tunneling, resistance, potential, conductivity, or lateral electrical measurement.

In some specific embodiments, the electrical signal is a current passing through the pore.

Preferably, the characterization further comprises applying a modified Viterbi algorithm.

A tenth aspect of the present application relates to use of the modified Prp43 helicase according to the first aspect of the present application or the protein construct according to the second aspect of the present application for characterizing a target polynucleotide or controlling movement of a target polynucleotide to pass through a pore.

### Sensor and Analysis Device

An eleventh aspect of the present application relates to an analysis device for characterizing a target polynucleotide, wherein the analysis device comprises one or more nanopores, one or more modified Prp43 helicases according to the first aspect of the present application or protein constructs according to the second aspect of the present application, and one or more containers.

Preferably, the analysis device is selected from a kit, a device, or a sensor.

Further preferably, the analysis device is a kit, wherein the kit comprises a chip comprising a lipid bilayer. The pores go across the lipid bilayer. The kit described herein comprises one or more lipid bilayers, wherein each lipid bilayer comprises one or more pores. The kit described herein further comprises a reagent or device for performing the characterization of the target polynucleotide. Preferably, the reagent includes a buffer and a tool required for PCR amplification.

A twelfth aspect of the present application relates to a method for forming a sensor for characterizing a target polynucleotide, which comprises providing a nanopore, and forming a complex between the nanopore and the modified Prp43 helicase according to the first aspect of the present application or the protein construct according to the second aspect of the present application.

Embodiments of the present application will be further explained and illustrated below with reference to the drawings and specific examples. These examples are intended only to explain and illustrate various aspects of the present application and should not be taken as limiting the scope of the present application.

### BRIEF DESCRIPTION OF THE DRAWINGS

FIG. 1 is a schematic diagram of a 3D structure of an N-terminus (M1-N60)-truncated wild-type Prp43 helicase (SEQ ID NO: 1) derived from *Chaetomium thermophilum.*
FIG. 2 shows the detection of single-stranded DNA-dependent ATP hydrolytic activity of an N-terminus (M1-N60)-truncated wild-type Prp43 helicase, a modified Prp43 helicase Prp43-2 (F181C/N623C/C508S), a modified Prp43 helicase Prp43-3 (P352C/S619C/C508S), an N-terminus (M1-N60)-truncated protein construct Prp43-GP, an N-terminus (M1-N60)-truncated protein construct Prp43-GP-2 (F181C/N623C/C508S), and an N-terminus (M1-N60)-truncated protein construct Prp43-GP-3 (P352C/S619C/C508S).

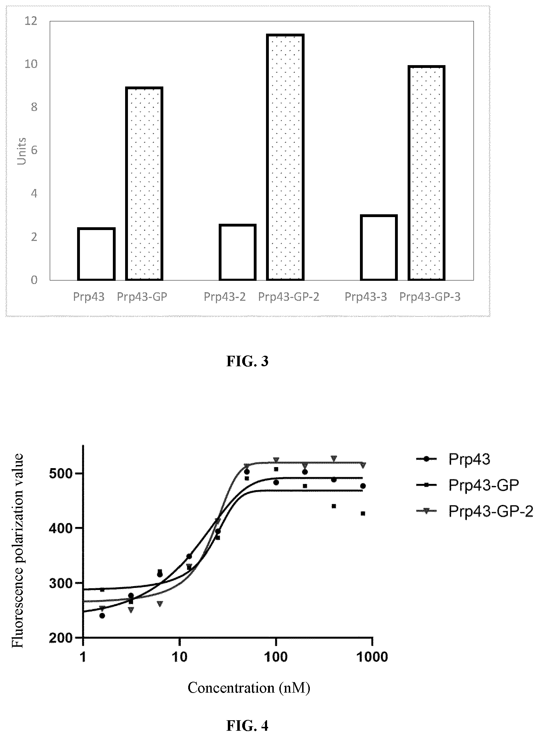

FIG. 3 shows the detection of single-stranded RNA-dependent ATP hydrolytic activity of an N-terminus (M1-N60)-truncated wild-type Prp43 helicase, a modified Prp43 helicase Prp43-2 (F181C/N623C/C508S), a modified Prp43 helicase Prp43-3 (P352C/S619C/C508S), an N-terminus (M1-N60)-truncated protein construct Prp43-GP, an N-terminus (M1-N60)-truncated protein construct Prp43-GP-2 (F181C/N623C/C508S), and an N-terminus (M1-N60)-truncated protein construct Prp43-GP-3 (P352C/S619C/C508S).
FIG. 4 shows curves of affinity of an N-terminus (M1-N60)-truncated wild-type Prp43 helicase, an N-terminus (M1-N60)-truncated protein construct Prp43-GP, or an N-terminus (M1-N60)-truncated protein construct Prp43-GP-2 (F181C/N623C/C508S) for single-stranded DNA under low salt conditions.

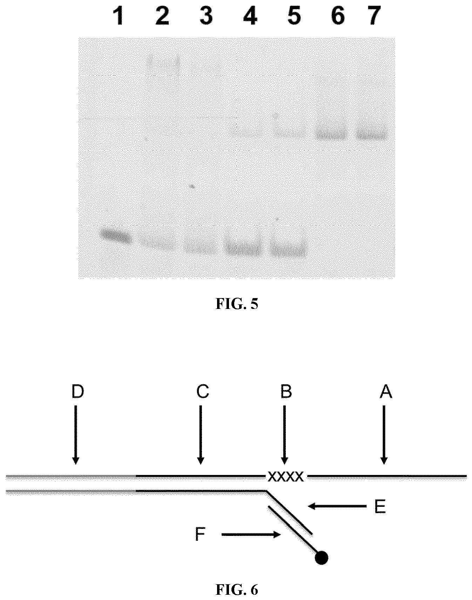

FIG. 5 shows the results of an electrophoresis mobility shift assay for an N-terminus (M1-N60)-truncated wild-type Prp43 helicase, an N-terminus (M1-N60)-truncated protein construct Prp43-GP, and an N-terminus (M1-N60)-truncated protein construct Prp43-GP-2 (F181C/N623C/C508S), in which lane 1 is a T44-37-FAM substrate, lane 2 is a complex in which a wild-type Prp43 helicase is bound to a T44-37-FAM substrate, lane 3 is a product in which a wild-type Prp43 helicase is bound to a T44-37-FAM substrate and then catalyzed with TMAD, lane 4 is a complex in which a Prp43-GP helicase is bound to a T44-37-FAM substrate, lane 5 is a product in which a Prp43-GP helicase is bound to a T44-37-FAM substrate and then catalyzed with TMAD, lane 6 is a complex in which a Prp43-GP-2 helicase mutant is bound to a T44-37-FAM substrate, and lane 7 is a product in which a Prp43-GP-2 helicase mutant is bound to a T44-37-FAM substrate and then catalyzed with TMAD.
FIG. 6 is a schematic diagram of a DNA construct X, in which the 5' end of a corresponding region A sequence SEQ ID NO: 32 is linked to a 4-iSpC3 spacer region (region B), which is linked to the 3' end of a corresponding region C sequence SEQ ID NO: 33, the 5' end of the region C sequence is linked to a corresponding region D sequence SEQ ID NO: 34, and a corresponding region E sequence SEQ ID NO: 35 of the construct is hybridized with a corresponding region F sequence SEQ ID NO: 36 (with a 3' cholesterol tether).

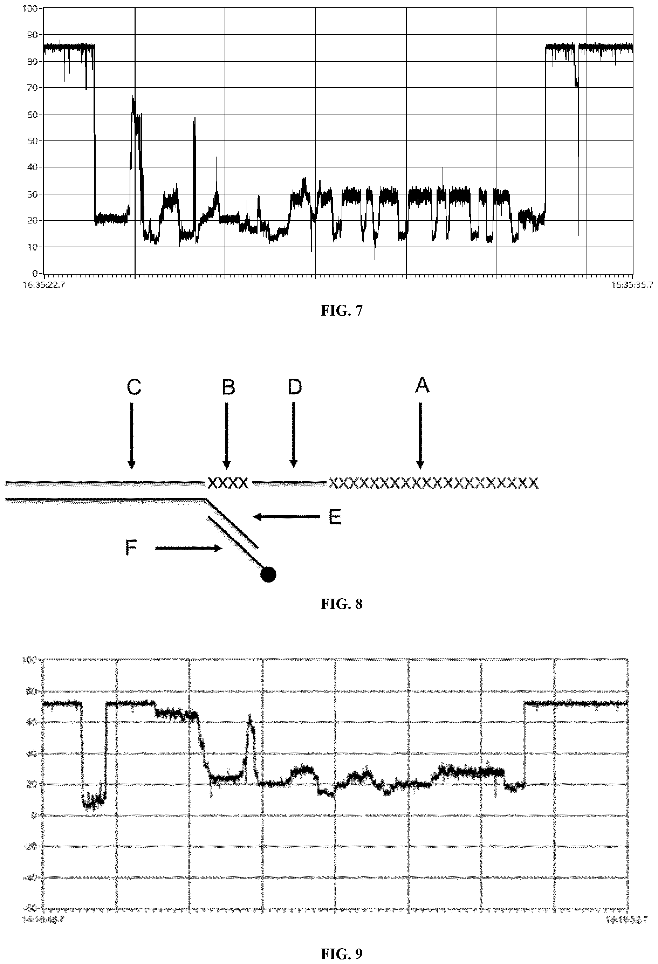

FIG. 7 shows an example of a current trajectory when the N-terminus (M1-N60)-truncated protein construct Prp43-GP-2 (F181C/N623C/C508S) controls movement of a DNA construct X to pass through an MspA nanopore (current (pA, 0 to 100) on the y-axis and time (h:m:s) on the x-axis).
FIG. 8 is a schematic diagram of an RNA construct, in which the 3' end of SEQ ID NO: 37 (denoted as D) is linked to a 20-iSpC3 spacer region (denoted as A), the 5' end is linked to a 4-iSpC3 spacer region (denoted as B), which is linked to the 3' end of SEQ ID NO: 38 (denoted as C), and a region of SEQ ID NO: 39 (denoted as E) of the construct is hybridized with SEQ ID NO: 40 (denoted as F, with a 3' cholesterol tether).
FIG. 9 shows an example of a current trajectory when the N-terminus (M1-N60)-truncated protein construct Prp43-GP-2 (F181C/N623C/C508S) controls an RNA construct Y to pass through an MspA nanopore (current (pA, 0 to 100) on the y-axis and time (h:m:s) on the x-axis).

### Examples

Details of the experimental procedures not specified in the examples below may be referred to references cited herein, and the experimental reagents and the instruments and equipment are conventional commercially available reagents and instruments and equipment.

### Example 1

A wild-type Prp43 helicase, a modified Prp43 helicase, and a protein construct were all prepared by standard molecular biology methods, the principles and procedures of which are well known to those skilled in the art (see references cited herein).

N-terminus-truncated wild-type Prp43 helicase (i.e., a T61-A764 fragment): a nucleic acid sequence (SEQ ID NO: 28, supplied by GenScript Biotech Corporation) corresponding to an N-terminus-truncated Prp43 helicase T61-A764 fragment (corresponding to an amino acid sequence of Prp43 helicase of SEQ ID NO: 1 with an M1-N60 fragment of the N-terminal domain removed) was ligated to a vector pGS-21a (GenScript Biotech Corporation, Cat. No. SD0121) by enzyme digestion and ligation, and transformed into an expression competent host cell BL21(DE3) (Beijing TransGen Biotech Co. Ltd., Cat. No. CD601-02) after being verified to be correct by sequencing. Monoclonal colonies were picked from a plate, seeded into 100 mL of an ampicillin-resistant liquid LB medium, cultured overnight at 37 °C, and transferred to a flask with a medium the next day for expansion. When OD600 reached about 0.4-0.8, isopropyl-β-D-thiogalactoside (IPTG) at a final concentration of 0.5 mM was added, and the expression was induced at 16 °C overnight for about 12 h. Bacteria collected by low-temperature centrifugation were resuspended in a lysis buffer and then crushed with a high-pressure homogenizer, and the supernatant was collected by high-speed centrifugation for subsequent purification by protein chromatography, specifically including nickel ion affinity chromatography, ion exchange chromatography, and molecular sieve separation. The target protein after removal of a GST tag by enzyme digestion was loaded on a nickel ion affinity chromatography column, and then the eluted target protein was collected. The target protein after the removal of the GST tag was assayed by SDS-PAGE gel electrophoresis. The truncated Prp43 protein (with M1-N60 of the N-terminal domain removed) after the removal of the tag was assayed by SDS-PAGE, which showed that the target protein was of the correct size and was available for subsequent testing and analysis.

Protein mutant Prp43-GP-2 (F181C/N623C/C508S) (i.e., SEQ ID NO: 27) of an N-terminus-truncated Prp43 helicase T61-A764 fragment fused to a GP domain: preparation was carried out according to the same preparation method as that for the N-terminus-truncated Prp43 helicase T61-A764 fragment, except that the initial nucleic acid sequence (SEQ ID NO: 28) corresponding to the N-terminus-truncated Prp43 helicase T61-A764 fragment, was replaced by SEQ ID NO: 30. The protein construct Prp43-GP-2 after the removal of the tag was assayed by SDS-PAGE, which showed that the target protein was of the correct size and was available for subsequent testing and analysis.

Modified N-terminus (M1-N60)-truncated Prp43 helicases and protein constructs were prepared according to the same method as that described above using different initial nucleic acid sequences: Prp43-2 (F181C/N623C/C508S), modified Prp43 helicase Prp43-3 (P352C/S619C/C508S), N-terminus (M1-N60)-truncated protein construct Prp43-GP, and N-terminus (M1-N60)-truncated protein construct Prp43-GP-3 (P352C/S619C/C508S). The initial nucleic acid sequences used are shown in Table 3 below.

**Table 3: proteins or protein constructs used in the examples and preparation thereof**

| Description | Abbreviation | Initial nucleic acid sequences for the preparation | Initial nucleic acid supplier |
|---|---|---|---|
| N-terminus-truncated wild-type Prp43 helicase | Prp43 | SEQ ID NO: 28 | GenScript Biotech Corporation |
| Modified N-terminus-truncated Prp43 helicase | Prp43-2 | SEQ ID NO: 41 | GenScript Biotech Corporation |
| (F181C/N623C/C508S) | | | |
| Modified N-terminus-truncated Prp43 helicase | Prp43-3 | SEQ ID NO: 42 | GenScript Biotech Corporation |
| (P352C/S619C/C508S) | | | |
| N-terminus-truncated Prp43 helicase protein construct | Prp43-GP | SEQ ID NO: 43 | GenScript Biotech Corporation |
| Modified N-terminus-truncated Prp43 helicase protein construct | Prp43-GP-2 | SEQ ID NO: 30 | GenScript Biotech Corporation |
| (F181C/N623C/C508S) | | | |
| Modified N-terminus-truncated Prp43 helicase protein construct | Prp43-GP-3 | SEQ ID NO: 44 | GenScript Biotech Corporation |
| (P352C/S619C/C508S) | | | |

### Example 2

In this example, the ATP hydrolytic activity of N-terminus (M1-N60)-truncated wild-type Prp43 helicase, modified Prp43 helicase Prp43-2 (F181C/N623C/C508S), modified Prp43 helicase Prp43-3 (P352C/S619C/C508S), N-terminus-truncated protein construct Prp43-GP, N-terminus-truncated protein construct Prp43-GP-2 (F181C/N623C/C508S), and N-terminus-truncated protein construct Prp43-GP-3 (P352C/S619C/C508S) when binding to or being incubated with a single-stranded DNA or single-stranded RNA substrate was tested.

### (1) Materials and methods

In this example, the ATPase hydrolytic activity of the Prp43 helicases was assayed by absorption photometry. Specifically, a premix solution containing 50 µM phosphate was prepared, wherein 50 µL of the phosphate standard solution was pipetted to 950 µL of ultrapure water, and the pipes were numbered.

**Table 4: preparation of standard**

| # | Premix + water | Final Vol (uL) | Phosphate Cone (uM) | pmoles Phosphate in 50 uL |
|---|---|---|---|---|
| 1 | 200 L + 0 L | 200 | 50 | 2,500 |
| 2 | 150 L + 50 L | 200 | 37.5 | 1875 |
| 3 | 125 L + 75 L | 200 | 31.25 | 1,562.5 |
| 4 | 100 L + 100 L | 200 | 25 | 1250 |
| 5 | 50 L + 150 L | 200 | 12.5 | 625 |
| 6 | 0 L + 200 L | 200 | 0 | 0 |

25 nM Prp43 helicase sample was added to duplicate wells of a 96-well plate, 0.5 nM M13ssDNA was added, and a test buffer (10 mM HEPES, 600 mM KCL, 5 mM Mg²⁺) was added to make a final volume of 10 µL. The mixture was reacted at 30 °C for 30 min. TMAD at a final concentration of 1 mM was added, and the resulting mixture was reacted at 30 °C for 30 min. 10 µL of buffer (10 mM HEPES, 50 mM KCL, 5 mM Mg²⁺) was added to duplicate wells as a negative control. High levels of phosphate can cause a background in the sample, which should be corrected. Immediately after the reaction mixture was added, 160 µL of working reagent was added to each background blank well to stop the reaction. The initial incubation for 30 min is not required, and then the background blank reading can be subtracted from the sample reading. The reaction combinations were set according to the schemes in Tables 4 and 5. 70 µL of the reaction mixture was required for the reaction of each sample, background blank, or negative control.

**Table 5: sample preparation**

| **Reagent** | **Sample, background blank, and negative control** |
|---|---|
| Test buffer (10 mM HEPES, 600 mM KCl, 5 mM Mg²⁺) | 66 uL |
| 4 mM ATP | 4 uL |

| | |
|---|---|
| 70 µL of the reaction mixture was added to each well, including blank background and negative control wells. | |

The reaction mixture was not added to the standards. The plate was incubated for reaction at room temperature for 30 min. 160 µL of working reagent was added to each well, and the plate was successively incubated at room temperature for 15 min. The enzymatic reaction was stopped and a colorimetric product was generated. The absorbance values at 600-660 nm [maximum absorbance at 620 nm (A620)] were read for all the samples, standards, and controls.

### (2) Results

The ATP hydrolytic activity of the N-terminus (M1-N60)-truncated wild-type Prp43 helicases and the modified Prp43 helicases or protein constructs after binding to DNA or RNA is shown in FIGs. 2 and 3. As can be seen from FIGs. 2 and 3, after a G-Path activator domain was fused to the C-terminus of the Prp43 helicases or mutants, the ATP hydrolytic activity of the helicases was significantly improved; after two cysteines were introduced into the Prp43 helicases, the ATP hydrolytic activity was also improved.

### Example 3

In this example, the affinity of the N-terminus (M1-N60)-truncated wild-type Prp43 helicases or the modified protein constructs Prp43-GP and Prp43-GP-2 (F181C/N623C/C508S) for single-stranded DNA was tested by fluorescence polarization.

### (1) Materials and methods

The N-terminus (M1-N60)-truncated wild-type helicases or modified helicases were diluted in the following concentration gradients: 800 nM, 400 nM, 200 nM, 100 nM, 50 nM, 25 nM, 12.5 nM, 6.25 nM, 3.125 nM,1.56 nM, and BLANK. The helicases were each incubated with 10 nM single-stranded DNA substrate for 20 min in a binding buffer (10 mM HEPES, 50 mM KCl, 5% Glycerol, pH 7.0), and read for a polarization value at 530 nM excitation wavelength and 560 nM emission wavelength, and affinity curves were fitted, with three replicates set for each helicase concentration.

### (2) Results

The fitting results are shown in FIG. 4, which showed that the affinity of the N-terminus (M1-N60)-truncated Prp43 helicase with a G-Path domain fused to the C-terminus, i.e., the Prp43-GP helicase, or the modified helicase Prp43-GP-2 (F181C/N623C/C508S) by site-directed mutagenesis based on Prp43-GP, for single-stranded DNA was not significantly different from that of the wild-type Prp43 helicase under low salt conditions.

### Example 4

In this example, the binding of the N-terminus (M1-N60)-truncated wild-type Prp43 helicases or the modified protein constructs Prp43-GP and Prp43-GP-2 (F181C/N623C/C508S) to DNA, including the enhancement of nucleic acid binding following disulfide bond formation between mutant sites F181C and N623C in the mutants catalyzed by a TMAD catalyst, was assayed by an electrophoresis mobility shift assay.

### (1) Materials and methods

The experimental procedures were as follows: 30 nM FAM fluorophore-labeled single-stranded poly-thymine substrate T44-37-FAM was added to a buffer (10 mM HEPES, 50 mM KCl, pH 7.0), the wild-type Prp43 helicase and the modified helicases Prp43-2 and Prp43-GP-2 at a final concentration of 120 nM were separately added, and the mixture was incubated at 30 °C for 1.5 h; a TMAD cross-linking agent at a final concentration 1000 times that of the helicase was used for catalyzing the crosslinking of cysteines at mutation sites, and the mixture was incubated at 30 °C for 1.5 h.

### (2) Results

The results of the electrophoresis mobility shift assay are shown in FIG. 5, which showed that the wild-type Prp43 helicase, after binding to DNA, had serious enzyme/nucleic acid dissociation under electrophoresis conditions, and the modified Prp43-GP helicase, after binding to DNA, had milder enzyme/nucleic acid dissociation under electrophoresis conditions than the wild-type Prp43 helicase, whereas the modified mutant Prp43-GP-2 had better binding to DNA, and no significant enzyme/nucleic acid dissociation was observed regardless of TMAD treatment.

### Example 5

In this example, the N-terminus (M1-N60)-truncated modified mutant helicase Prp43-GP-2 (F181C/N623C/C508S) controlled movement of a DNA construct X to pass through an MspA nanopore.

### (1) Materials and methods

Preparation of a DNA construct X as shown in FIG. 6: the 5' end of a corresponding region A sequence (SEQ ID NO: 32) was linked to a 4-iSpC3 spacer region (region B), which was linked to the 3' end of a corresponding region C sequence (SEQ ID NO: 33), the 5' end of the region C sequence was linked to a corresponding region D sequence (SEQ ID NO: 34), and a corresponding region E sequence (SEQ ID NO: 35) of the construct was hybridized with a corresponding region F sequence (SEQ ID NO: 36, with a 3' cholesterol tether). The synthetically linked fragments A, B, C, and D at a concentration of 10 µM and the fragments E and F were added to an annealing buffer (10 mM Tris, pH 7.0, 50 mM NaCl) according to a ratio of 1:1:1, and the mixture was annealed according to the following procedures: 98 °C for 10 min, -0.1 °C/0.6 s, 300 cycles; 65 °C for 5 min, - 0.1 °C/0.6 s, 400 cycles (fragments A, B, C, D, E, and F were supplied by Sangon Biotech (Shanghai) Co., Ltd.). The prepared DNA construct X and the modified mutant helicase Prp43-GP-2 (F181C/N623C/C508S) or N-terminus-truncated wild-type Prp43-GP were pre-incubated in a buffer (10 mM HEPES, pH 8.0, 50 mM NaCl, 5% glycerol) at 25 °C for 30 min, a TMAD catalyst at a concentration 1000 times that of the helicase was added, and the mixture was incubated at room temperature for 30 min. Electrical measurement signals were obtained from MspA nanopores (MspA protein sequence of SEQ ID NO: 31, prepared as described in Michael Faller et al., "The Structure of a Mycobacterial Outer-Membrane Channel", Science 303, 1189 (2004); DOI: 10.1126/science.1094114) embedded in the 1,2-diethanoyl-glycero-3-phosphocholine lipid bilayer. By the Montal-Mueller technique, a bilayer was formed in a pore with a diameter of about 25 µm on a PTFE membrane, and thus two buffer solutions of about 100 µL were separated. All experiments were performed in the buffer. The single-channel current was measured using an amplifier equipped with a digitizer. The Ag/AgCl electrode was connected to the buffer, such that a cis-compartment was connected to the ground end of the amplifier and a trans-compartment was connected to the active electrode.

After a single pore is formed on the bilayer, a complex of the DNA polynucleotide and the modified mutant helicase Prp43-GP-2 (F181C/N623C/C508S) or N-terminus-truncated wild-type Prp43-GP was added to 70 µL of buffer in the cis-compartment of the electrophysiology chamber to trigger the capture of the helicase-DNA complex in the nanopore. The ATPase activity of the helicase was activated by adding a divalent metal (5 mM MgCl₂) and NTP (2.86 µM ATP) to the cis-compartment as required. The experiment was performed at a constant potential of +180 mV.

### (2) Results

The results showed that the movement of the DNA construct X was controlled by the helicase Prp43-GP-2 (F181C/N623C/C508S). As shown in FIG. 7, the results showed that the Prp43-GP-2 helicase controlled translocation of a DNA construct of approximately 200 bp to pass through the nanopore. Correspondingly, the N-terminus-truncated wild-type Prp43 (T61-A764 fragment) or construct Prp43-GP made it difficult to obtain the sustained current signals generated by the fragment A/B/C/D of the construct X passing through the nanopore.

### Example 6

In this example, the N-terminus (M1-N60)-truncated modified mutant helicase Prp43-GP-2 (F181C/N623C/C508S) controlled movement of an RNA construct Y to pass through an MspA nanopore.

### (1) Materials and methods

Preparation of an RNA construct as shown in FIG. 8: the 3' end of a corresponding region D sequence (SEQ ID NO: 37) was linked to a 20-iSpC3 spacer region (region A), the 5' end of which was linked to a 4-iSpC3 spacer region (region B), which was linked to the 3' end of a corresponding region C sequence (SEQ ID NO: 38), and a corresponding region E sequence (SEQ ID NO: 39) of the construct was hybridized with a corresponding region F sequence (SEQ ID NO: 40). The synthetically linked fragments A, B, C, and D at a concentration of 10 µM and the fragments E and F were added to an annealing buffer (10 mM Tris, pH 7.0, 50 mM NaCl) according to a ratio of 1:1:1, and the mixture was annealed according to the following procedures: 98 °C for 10 min, - 0.1 °C/0.6 s, 300 cycles; 65 °C for 5 min, -0.1 °C/0.6 s, 400 cycles (fragments A, B, C, D, E, and F were supplied by Sangon Biotech (Shanghai) Co., Ltd.).

The prepared RNA construct and the helicase Prp43-GP-2 or N-terminus-truncated wild-type Prp43-GP were pre-incubated in a buffer (10 mM HEPES, pH 7.0, 50 mM NaCl) at 30 °C for 30 min. Electrical measurement signals were obtained from MspA nanopores (MspA protein sequence of SEQ ID NO: 31, prepared as described in Michael Faller et al., "The Structure of a Mycobacterial Outer-Membrane Channel", Science 303, 1189 (2004); DOI: 10.1126/science.1094114) embedded in the 1,2-diethanoyl-glycero-3-phosphocholine lipid bilayer. By the Montal-Mueller technique, a bilayer was formed in a pore with a diameter of about 25 µm on a PTFE membrane, and thus two buffer solutions of about 100 µL were separated. All experiments were performed in the buffer. The single-channel current was measured using an amplifier equipped with a digitizer. The Ag/AgCl electrode was connected to the buffer, such that a cis-compartinent was connected to the ground end of the amplifier and a trans-compartment was connected to the active electrode.

After a single pore is formed on the bilayer, the RNA polynucleotide construct and the Prp43-GP-2 helicase or N-terminus-truncated wild-type Prp43-GP were added to 70 µL of buffer in the cis-compartment of the electrophysiology chamber to trigger the capture of the helicase-RNA complex in the nanopore. The ATPase activity of the helicase was activated by adding a divalent metal (5 mM MgCl₂) and NTP (5 mM ATP) to the cis-compartment as required. The experiment was performed at a constant potential of +180 mV.

### (2) Results

The results showed that the movement of the RNA construct was controlled by the Prp43-GP-2 helicase. The results for the control of the movement of the RNA by the Prp43-GP-2 helicase are shown in FIG. 9. The Prp43-GP-2 helicase controlled the movement of the RNA for 3 s, which corresponds to translocation of an RNA construct of approximately 30 bp to pass through the nanopore. Correspondingly, the N-terminus-truncated wild-type Prp43 (T61-A764 fragment) or N-terminus-truncated construct Prp43-GP made it difficult to obtain the sustained current signals generated by the fragment A/B/C/D of the construct Y passing through the nanopore.

Preferred embodiments and specific examples of the present invention are described herein, but these embodiments and examples are provided by way of illustration only, and are not intended to limit the present invention. Numerous variations, changes and substitutions will occur to those skilled in the art without departing from the present invention. Accordingly, the present invention shall also encompass any such alternatives, modifications, variations, or equivalents.

## Claims

1. A modified Prp43 helicase, comprising a RecA 1 domain, a RecA2 domain, and a Ratchet domain, wherein the modified Prp43 helicase comprises insertion or replacement of 1, 2, 3, 4, 5, 6, 7, 8, 9, 10, 11, or more cysteines and/or insertion or replacement of 1, 2, 3, 4, 5, 6, 7, 8, 9, 10, 11, or more non-natural amino acids introduced into at least one domain selected from the RecA1 domain, the RecA2 domain, or the Ratchet domain, relative to a corresponding wild-type Prp43 helicase or a fragment thereof.

2. The modified Prp43 helicase according to claim 1, wherein the introduced cysteine residues or non-natural amino acid residues are located at positions corresponding to any one or two or more of M157, Q161, D165, F181, E182, N183, R324, L328, E332, R335, P353, L351, P352, H354, D321, E320, R358, P563, A564, N565, D603, K605, K606, H609, Y615, R616, S619, N623, A626, or K630 of SEQ ID NO: 1, preferably at positions corresponding to any one or two or more of F181, P352, S619, or N623 of SEQ ID NO: 1.

3. The modified Prp43 helicase according to claim 1 or 2, wherein the fragment of the wild-type Prp43 helicase is a fragment obtained after removal of an N-terminal domain of the Prp43 helicase, preferably removal of at least 96, at least 90, at least 80, at least 70, at least 60, at least 50, at least 40, or at least 30 residues beginning at position 1 of the N-terminus.

4. The modified Prp43 helicase according to any one of claims 1 to 3, wherein the modified Prp43 helicase further comprises replacement of one or more cysteine residues, preferably replacement of one or more cysteine residues corresponding to C148, C214, C303, C323, C377, C441, C508, C543, or C608 of SEQ ID NO: 1, and more preferably replacement of cysteine residues with an alanine, glycine, valine, isoleucine, leucine, phenylalanine, tyrosine, serine, threonine, aspartic acid, glutamic acid, lysine, arginine, histidine, methionine, tryptophan, glutamine, asparagine, or proline residue.

5. The modified Prp43 helicase according to any one of claims 1 to 4, wherein the total number of the introduced cysteine residues and non-natural amino acid residues is 2 or more, and an interconnection is formed between at least one introduced cysteine or non-natural amino acid residue and another introduced cysteine or non-natural amino acid residue.

6. The modified Prp43 helicase according to claim 5, wherein the connection is selected from covalent connection, hydrogen bonding connection, electrostatic interaction, π-π interaction, or hydrophobic interaction, preferably covalent connection.

7. The modified Prp43 helicase according to claim 6, wherein the covalent connection is a covalent connection achieved by a -S-S bond or by a cross-linking agent or catalyst selected from phosgene, maleimide, active ester, succinimide, azide, alkane, alkene, alkyne, polyethylene glycol (PEG), polypeptide, polysaccharide, deoxyribonucleic acid (DNA), peptide nucleic acid (PNA), threose nucleic acid (TNA), glycerol nucleic acid (GNA), polyamide, or TMAD.

8. The modified Prp43 helicase according to any one of claims 1 to 7, wherein the modified Prp43 helicase further comprises one or more amino acid modifications selected from the group consisting of:
(a) replacement of one or more amino acids that interact with nucleotides;
(b) replacement of one or more amino acids associated with binding of NTP and/or a divalent metal ion;
(c) replacement of one or more amino acids that interact with transmembrane pores; and
(d) further modification to reduce a negative charge on the surface of the Prp43 helicase.

9. The modified Prp43 helicase according to any one of claims 1 to 8, wherein the modified Prp43 helicase is derived from *Chaetomium thermophilum, Bathycoccus prasinos, Uncultured bacterium, Archaeon, Parcubacteria, Sorangium cellulosum, Candidatus Sungbacteria, Mycolicibacterium chitae, Parcubacteria, Thermodesulforhabdus norvegica, Deltaproteobacteria, Puniceicoccales, Desulfobacterium vacuolatum* or *Desulfobacter sp.,* or derived from a viral metagenome.

10. The modified Prp43 helicase according to any one of claims 1 to 8, wherein the wild-type Prp43 helicase is a Prp43 helicase selected from a Prp43 helicase having one of the following sequences: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, or SEQ ID NO: 15.

11. The modified Prp43 helicase according to any one of claims 1 to 8, wherein the modified Prp43 helicase has at least 30%, at least 40%, at least 50%, at least 60%, at least 70%, at least 80%, at least 85%, at least 90%, at least 91%, at least 92%, at least 93%, at least 94%, at least 95%, at least 96%, at least 97%, at least 98%, at least 99%, or at least 99.9% homology to the amino acid sequence of the corresponding wild-type Prp43 helicase.

12. The modified Prp43 helicase according to any one of claims 1 to 8, wherein the modified Prp43 helicase is derived from *Chaetomium thermophilum,* and preferably, the modified Prp43 helicase has at least 30%, at least 40%, at least 50%, at least 60%, at least 70%, at least 80%, at least 85%, at least 90%, at least 91%, at least 92%, at least 93%, at least 94%, at least 95%, at least 96%, at least 97%, at least 98%, at least 99%, or at least 99.9% homology to the amino acid sequence of SEQ ID NO: 1.

13. The modified Prp43 helicase according to any one of claims 1 to 3, wherein the modified Prp43 helicase is derived from *Chaetomium thermophilum,* and the introduced cysteine residues or non-natural amino acid residues are located at positions corresponding to any one or more of F181, P352, S619, or N623 of SEQ ID NO: 1.

14. The modified Prp43 helicase according to claim 13, wherein the modified Prp43 helicase is a modified T61-A764 fragment of SEQ ID NO: 1, and the modification is selected from F181C/N623C/C508S and P352C/S619C/C508S.

15. The modified Prp43 helicase according to any one of claims 1 to 14, wherein the modified Prp43 helicase is in the form of an oligomer comprising one or more said modified Prp43 helicases according to any one of claims 1 to 12.

16. A protein construct, comprising the modified Prp43 helicase according to any one of claims 1 to 15, and a G-Path domain of an auxiliary activator protein Pfal or a fragment of Pfal containing the G-Path domain fused to the C-terminus or N-terminus of the Prp43 helicase.

17. The protein construct according to claim 16, wherein the protein construct comprises one or more said modified Prp43 helicases.

18. The protein construct according to claim 16 or 17, wherein the auxiliary activator protein Pfal is Pfal derived from *Chaetomium thermophilum var. thermophilum, Thermothielavioides terrestris, Thermothelomyces thermophilus*, *Podospora anserina*, *Neurospora tetrasperma*, *Coniochaeta sp*., *Monosporascus sp*., *Hypoxylon sp*., *Madurella mycetomatis*, or *Coniochaeta pulveracea.*

19. The protein construct according to claim 16 or 17, wherein the amino acid sequence of the auxiliary activator protein Pfa1 is selected from an amino acid sequence of SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, or SEQ ID NO: 25, or an amino acid sequence of a variant having at least 30%, at least 40%, at least 50%, at least 60%, at least 70%, at least 80%, at least 85%, at least 90%, at least 91%, at least 92%, at least 93%, at least 94%, at least 95%, at least 96%, at least 97%, at least 98%, at least 99%, or at least 99.9% homology to the amino acid sequence of one of SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, or SEQ ID NO: 25, and the auxiliary activator protein Pfal has a function of activating the Prp43 helicase.

20. The protein construct according to claim 16 or 17, wherein the G-Path domain of Pfa1 is a K662-G742 fragment (SEQ ID NO: 26) of the sequence of SEQ ID NO: 16, or an amino acid sequence of a variant having at least 30%, at least 40%, at least 50%, at least 60%, at least 70%, at least 80%, at least 85%, at least 90%, at least 91%, at least 92%, at least 93%, at least 94%, at least 95%, at least 96%, at least 97%, at least 98%, at least 99%, or at least 99.9% homology to the amino acid sequence of SEQ ID NO: 26, and the variant has a function of activating the Prp43 helicase.

21. The protein construct according to claim 16, wherein the Prp43 helicase is a T61-A764 fragment of SEQ ID NO: 1, and has insertion or replacement of 1 or more cysteines and/or insertion or replacement of non-natural amino acids introduced at positions corresponding to any one or two or more of F181, P352, S619, or N623 of SEQ ID NO: 1, and the amino acid sequence of the auxiliary activator protein Pfa1 is SEQ ID NO: 16.

22. The protein construct according to claim 16, wherein the Prp43 helicase is T61-A764 of SEQ ID NO: 1, and further has a modification selected from F181C/N623C/C508S and P352C/S619C/C508S, and the C-terminus of the Prp43 helicase is fused to a polypeptide having an amino acid sequence of SEQ ID NO: 26.

23. A nucleic acid, encoding the modified Prp43 helicase according to any one of claims 1 to 15 or the protein construct according to any one of claims 16 to 22.

24. The nucleic acid according to claim 23, wherein the nucleic acid is comprised in a vector selected from a plasmid, a virus, and a phage.

25. An expression vector, comprising the nucleic acid according to claim 23.

26. The expression vector according to claim 25, wherein the expression vector is selected from a plasmid, a virus, and a phage.

27. The expression vector according to claim 25 or 26, wherein the expression vector further comprises a regulatory element for controlling expression of the nucleic acid.

28. The expression vector according to claim 27, wherein the regulatory element is a promoter operably linked to the nucleic acid.

29. The expression vector according to claim 28, wherein the promoter is selected from T7, trc, lac, ara, and µL.

30. A host cell, comprising the nucleic acid according to claim 23 or 24 or comprising the expression vector according to any one of claims 25 to 28.

31. The host cell according to claim 30, wherein the host cell is *Escherichia coli.*

32. A method for preparing the protein construct according to any one of claims 16 to 22, comprising: providing a polypeptide of SEQ ID NO: 1 or a variant thereof and a polypeptide of SEQ ID NO: 26 or a variant thereof, introducing at least one cysteine residue and/or at least one non-natural amino acid into the polypeptide of SEQ ID NO: 1 or the variant thereof, and fusing the polypeptide of SEQ ID NO: 26 or the variant thereof to the C-terminus or N-terminus of the resulting polypeptide to form the protein construct.

33. A method for preparing the modified Prp43 helicase according to any one of claims 1 to 15 or the protein construct according to any one of claims 16 to 22, comprising: culturing the host cell according to claim 30 or 31, inducing expression, and purifying the resulting expression product.

34. A method for controlling movement of a polynucleotide molecule, comprising contacting the polynucleotide molecule with the modified Prp43 helicase according to any one of claims 1 to 15 or the protein construct according to any one of claims 16 to 22.

35. The method for controlling movement of a polynucleotide molecule according to claim 34, wherein the polynucleotide molecule is controlled to pass through a nanopore, and the nanopore is a transmembrane pore.

36. The method for controlling movement of a polynucleotide molecule according to claim 35, wherein the transmembrane pore is selected from a protein pore, a solid pore, or a pore in which a biological pore is hybridized with a solid pore, and preferably, the protein pore is selected from *Mycobacterium smegmatis* porin *A, Mycobacterium smegmatis* porin B, *Mycobacterium smegmatis* porin C, *Mycobacterium smegmatis* porin D, hemolysin, lysenin, interleukin, outer membrane porin F, outer membrane porin G, outer membrane phospholipase A, WZA, or *Neisseria* autotransporter lipoprotein.

37. A method for characterizing a target polynucleotide, comprising:
(a) contacting the target polynucleotide with the modified Prp43 helicase according to any one of claims 1 to 15 or the protein construct according to any one of claims 16 to 22, such that the Prp43 helicase or protein construct controls movement of the target polynucleotide to pass through a nanopore;
(b) acquiring one or more characteristics of nucleotides in the target polynucleotide when interacting with the nanopore, thereby characterizing the target polynucleotide.

38. The method for characterizing a target polynucleotide according to claim 37, wherein the method further comprises the step of applying a potential difference across the nanopore.

39. The method for characterizing a target polynucleotide according to claim 37 or 38, wherein one or more said Prp43 helicases or protein constructs are used in the method.

40. The method for characterizing a target polynucleotide according to claim 37 or 38, wherein the nanopore is a transmembrane pore selected from a protein pore, a solid pore, or a pore in which a biological pore is hybridized with a solid pore, and preferably, the protein pore is selected from *Mycobacterium smegmatis* porin *A, Mycobacterium smegmatis* porin B, *Mycobacterium smegmatis* porin C, *Mycobacterium smegmatis* porin D, hemolysin, lysenin, interleukin, outer membrane porin F, outer membrane porin G, outer membrane phospholipase A, WZA, or *Neisseria* autotransporter lipoprotein.

41. Use of the modified Prp43 helicase according to any one of claims 1 to 15 or the protein construct according to any one of claims 16 to 22 for characterizing a target polynucleotide or controlling movement of a target polynucleotide to pass through a pore.

42. An analysis device for characterizing a target polynucleotide, comprising one or more nanopores, one or more said modified Prp43 helicases according to any one of claims 1 to 15 or said protein constructs according to any one of claims 16 to 22, and one or more containers.

43. The analysis device for characterizing a target polynucleotide according to claim 42, wherein the analysis device further comprises a chip comprising a lipid bilayer, and the nanopores go across the lipid bilayer.

44. The analysis device for characterizing a target polynucleotide according to claim 42 or 43, wherein the analysis device further comprises a buffer and a PCR amplification reagent.

45. The analysis device for characterizing a target polynucleotide according to claim 42, 43, or 44, wherein the analysis device is a kit or a sensor.

46. A method for forming a sensor for characterizing a target polynucleotide, comprising providing a nanopore, and forming a complex between the nanopore and the modified Prp43 helicase according to any one of claims 1 to 15 or the protein construct according to any one of claims 16 to 22.
